# EUROPEAN PATENT APPLICATION

(11) **EP 3 412 288 A1**
(43) Date of publication of application: **12.12.2018**
(21) Application number: 17305679.7
(22) Date of filing: 08.06.2017
(51) Int. Cl.: A61K 31/395, A61K 31/495, A61P 17/10

(54) **VEGF INHIBITORS FOR USE FOR PREVENTING AND/OR TREATING ACNE**

(71) Applicant: Galderma Research & Development, 06410 Biot (FR); Agency for Science, Technology and Research, Singapore 138632 (SG)
(72) Inventor: VIAL, Emmanuel, 06300 NICE (FR); OUVRY, Gilles, 06410 BIOT (FR); HACINI-RACHINEL, Fériel, 06410 BIOT (FR); GINHOUX, Florent, 278178 Singapore (SG); JANELA, Baptiste, 138642 Singapore (SG)
(74) Representative: Lavoix

(57) **Abstract**

The present invention relates to the use of a VEGF inhibitor for use for preventing and/or treating acne.

## Description

The present invention concerns the use of VEGF inhibitors for preventing and/or treating acne.

Acne is a common multi-factor pathology that attacks skin rich in sebaceous glands (face, shoulder area, arms and intertriginous areas). It is the most commonly occurring form of dermatosis. The following five pathogenic factors play a determining role in the development of acne:
1. genetic predisposition;
2. overproduction of sebum (seborrhoea);
3. androgens;
4. follicular keratinization disorders (comedogenesis); and
5. inflammatory factors and follicular colonization by the gram-positive bacterium, *Propionibacterium acnes* (*P. acnes*) (Zouboulis, 2005, Bojar and Holland, 2004). *P. acnes* is a commensal of the human skin, mouth and upper respiratory tract (Cogen et al., 2008, Costello et al., 2009, Fierer et al., 2008, Gao et al., 2007) but is also an opportunistic pathogen (Fitz-Gibbon et al., 2013).

There are several forms of acne, the common factor of all of them being the attack of the pilosebaceous follicles. Exemplary are acne conglobata, acne keloid on the nape of the neck, acne medicamentosa, recurrent miliary acne, acne necrotica, acne neonatorum, premenstrual acne, occupational acne, acne rosacea, senile acne, solar acne and acne vulgaris.

Acne vulgaris, also known as polymorphous juvenile acne, is the most common. It comprises four stages, but it is not necessary to pass through all the stages:
- Stage 1 corresponds to comedonal acne, characterized by a large number of open and/or closed comedones and of microcysts;
- Stage 2, or papulopustular acne, is of mild to moderate seriousness. It is characterized by the presence of open and/or closed comedones and microcysts, but also of red papules and of pustules. It mainly affects the face and leaves few scars;
- Stage 3, or papulocomedonal acne, is more serious and extends to the back, the thorax and the shoulders. It is accompanied by a larger number of scars; and
- Stage 4, or nodulocystic acne, is accompanied by numerous scars. It exhibits nodules and also has large painful purplish pustules.

The various forms of acne described above can be treated with active agents, such as antiseborrhoeics and antiinfectives, for example benzoyl peroxide (in particular, the product Eclaran® marketed by Pierre Fabre); with retinoids, such as tretinoin (in particular, the product Retacnyl® marketed by Galderma) or isotretinoin (the product Roaccutane® marketed by Laboratoires Roche); or with naphthoic acid compounds, such as, in particular, 6-[3-(1-adamantyl)-4-methoxyphenyl]-2-naphthoic acid, commonly known as adapalene (the product Differine® marketed by Galderma).

Combinations of several local treatments (such as antibiotics, retinoids, peroxides or zinc) is also used in dermatology to increase the efficacy of the active ingredients and to reduce their toxicity, but the multiple application of various dermatological products can be quite laborious and demanding for the patient.

There is thus still a need for efficient acne treatments, which may preferably be administered via the topical route.

Among the five pathogenic factors involved in acne, the inflammatory response to *P.acnes* bacterium drives the development of acne lesions: significantly higher numbers of T lymphocytes (Kistowska et al., 2015) and neutrophils accumulate in acne patient skin and around lesions, where they can promote breakdown of the follicular wall, stimulating further inflammation, likely through the production of reactive-oxygen species (Akamatsu et al., 2003).
Similarly, in rodents, injection of *P. acnes* into the skin results in recruitment of the polymorphonuclear cells, macrophages and T lymphocytes (De Young et al., 1984).

It results that limiting and/or avoiding the inflammatory response to *P.acnes* bacterium would help controlling the development of acne lesions.

The present invention now offers a treatment for reaching this purpose, and offers an improved therapy for acne, preferably via the topical route.

Surprisingly, the inventors have discovered that, in a murine model in which *P. acnes* was injected into the skin, successive waves of immune cell infiltrates over 30 days, and that conventional dendritic cells subset 1 (cDC1) are key mediators of neutrophil recruitment, phenotype, survival and function following *P. acnes* exposure. The inventors further identified that a minor subset of EpCAM⁺CD59⁺Ly6D⁺ cDC1 critically secrete VEGFα (also called VEGF-A), which is responsible for the observed effects via the control of the expression of the receptor VEGF-R1 on neutrophils. Finally, after concomitant intradermal injection of WT mice with anti-VEGFα antibodies and *P. acnes,* significant reductions in ear swelling and neutrophil infiltration, thus in inflammation, were observed compared to control WT mice.
Altogether, it results that the secretion of VEGFα by the minor subset of cDC1 is essential in the regulation of neutrophil biology, and represents a target of choice.

Thus, the present invention aims to use VEGF inhibitors for preventing and/or treating acne.
Indeed, targeting VEGF and inhibiting its expression, activity and/or signaling pathway, such as via the topical route (i.e. on skin), would allow controlling, and thereby reducing, the recruitment of neutrophils, thus preventing and/or decreasing the inflammatory response to *P.acnes* bacterium, and thereby the development of acne lesions.

The invention thus relates to a VEGF inhibitor for use for preventing and/or treating acne, preferably via the topical route.
The invention further concerns a method of preventing and/or treating acne, comprising administering to a patient in need thereof a therapeutically effective amount of a VEGF inhibitor.

By acne it is understood, all acne forms especially simple acne, comedogenic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne, medication-related acne and acne vulgaris.
Preferably, the invention is intended for preventing and/or treating acne vulgaris.

Preferably, the VEGF inhibitor is used for preventing and/or treating acne, by reducing the recruitment of neutrophils. Preferably, the VEGF inhibitor prevents and/or decreases the inflammatory response to *P.acnes* bacterium. Preferably, the VEGF inhibitor prevents and/or decreases the development of acne lesions. Preferably, the VEGF inhibitor prevents and/or decreases VEGF-A-signalling on neutrophils induced by cDC1, particularly by the subset of EpCAM⁺CD59⁺Ly6D⁺ cDC1.
By "VEGF-A-signalling on neutrophils", it is meant VEGF-R1 expression on neutrophils.

The subset of EpCAM⁺CD59⁺Ly6D⁺ cDC1 is a subset of dendritic cells.
Dendritic cells (DC) are professional pathogen-sensing and antigen-presenting cells (APC) that are central to the initiation and regulation of immune responses (Schlitzer et al., 2015). Conventional dendritic cells (cDCs) are innate immune cells. The term cDC refers to all DCs other than plasmacytoid DCs. In the skin, two subsets of ontogenetically-distinct and functionally-specialized conventional DC (cDC) exist: cDC1, identified in mice as expressing CD103 (integrin αE); and cDC2, expressing the CD11b (integrin αM), and SIRPα (Schlitzer and Ginhoux, 2014, Schlitzer et al., 2015, Guilliams et al., 2016).
Within the epidermis there are also Langerhans cells (LC), whose surface phenotype partially overlaps with both skin cDC (MHCII, CD207 (langerin), CD24 and CD11c) and macrophages (F4/80) (Ginhoux and Merad, 2010, Guilliams et al., 2016); however LC arise from a distinct developmental pathway and possess unique functional features (Romani et al., 2010).
Together, these APC populations sense and integrate multiple signals from the internal and external environments in order to initiate and shape optimal immune responses.
By "subset of EpCAM⁺CD59⁺Ly6D⁺ cDC1 ", it is meant the subset of cDC which may be present in skin and which express the surface markers CD103 (or integrin αE), EpCAM (epithelial cell adhesion molecule), CD59 (or MAC-inhibitory protein or membrane inhibitor of reactive lysis or MIRL) and Ly6D (or lymphocyte antigen 6 complex, locus D; or E48).

VEGF (Vascular Endothelial Growth Factor) is a well-known signal protein produced by cells that stimulates vasculogenesis and angiogenesis. In mammals, the VEGF family comprises 5 members: VEGF-A (or VEGFα), placenta growth factor, VEGF-B, VEGF-C and VEGF-D.
All members of the VEGF family stimulate cellular responses by binding to tyrosine kinase receptors (the VEGFRs) on the cell surface, causing them to dimerize and become activated through transphosphorylation. The VEGF receptors have an extracellular portion consisting of 7 immunoglobulin-like domains, a single transmembrane spanning region, and an intracellular portion containing a tyrosine-kinase domain. VEGF-A binds to VEGFR-1 and VEGFR-2. VEGF-C and VEGF-D, but not VEGF-A, are ligands for a third receptor (VEGFR-3), which mediates lymphangiogenesis.

By "VEGF inhibitor" according to the present invention, it is meant any compound that inhibits or reduces VEGF biological activity. The biological activity of VEGF depends on the amount of the VEGF nucleic acid (i.e. its expression level) and/or on the amount of the VEGF protein; on VEGF interaction with its target sequences, particularly its receptor(s) (called VEGFR); or on the activation of VEGFR.
Therefore, the VEGF inhibitor may reduce or inhibit VEGF expression, or reduce or inhibit VEGF interaction ability with its target sequences, particularly its receptor(s), or reduce or inhibit the activation of VEGFR. Preferably, the VEGF inhibitor is a VEGF-A inhibitor.
"VEGF expression" refers to events modifying VEGF mRNA transcriptionally or post-transcriptionally, by cleavage and maturation, to provide a functional VEGF, notably any reaction which results in inhibition of VEGF mRNA processing; it also includes events modifying VEGF protein during translation, as well as post-translational modifications. As used herein, the term "target sequence" of VEGF according to the invention is a sequence to which VEGF specifically binds. Preferably, the target sequence is VEGF-R1, VEGF-R2 or VEGF-R3, more preferably VEGF-R1.

An "inhibitor of VEGF expression" refers to any compound that has a biological effect to inhibit the expression of a VEGF gene and/or the expression of a VEGF protein.
In one embodiment of the invention, said inhibitor of VEGF gene expression is a siRNA, or an antisense oligonucleotide.
Small inhibitory RNAs (siRNAs) can function as inhibitors of gene expression for use in the invention. Gene expression can be reduced with a small double stranded RNA (dsRNA), or a vector or construct causing the production of a small double stranded RNA, such that gene expression is specifically inhibited (i.e. RNA interference or RNAi). Methods for selecting an appropriate dsRNA or dsRNA-encoding vector are well known in the art for genes whose sequence is known (e.g. see U.S. Pat. Nos. 6,573,099 and 6,506,559; International Patent Nos. WO 01/36646, WO 99/32619, and WO 01/68836). Inhibitors of VEGF for use in the invention may be based on antisense oligonucleotide (ODNs) constructs. Antisense oligonucleotides, including antisense RNA molecules and antisense DNA molecules, would act to directly block the activity of VEGF by binding to VEGF mRNA and thus preventing binding leading to mRNA degradation.
For example, antisense oligonucleotides of at least about 15 bases and complementary to unique regions of the VEGF transcript sequence can be synthesized, e.g., by conventional phosphodiester techniques and administered by e.g., intravenous injection or infusion. Methods for using antisense techniques for specifically inhibiting gene expression of genes whose sequence is known are well known in the art (e.g. see U.S. Pat. Nos. 6,566,135; 6,566,131; 6,365,354; 6,410,323; 6,107,091; 6,046,321; and 5,981,732). It should be further noted that antisense oligonucleotides may be modified with phosphorothioate to prevent their in vivo hydrolysis by nucleases. Such modifications are well known in the art. Antisense oligonucleotides useful as inhibitors of VEGF can be prepared by known methods. These include techniques for chemical synthesis such as, e.g., by solid phase phosphoramadite chemical synthesis. They can also be generated by in vitro or in vivo transcription of DNA sequences encoding the RNA molecule. Such DNA sequences can be incorporated into a wide variety of vectors that incorporate suitable RNA polymerase promoters such as the T7 or SP6 polymerase promoters.

As used herein, the terms "inhibitor of the interaction" means preventing or reducing the direct or indirect association of one or more molecules, nucleic acids, peptides, proteins. As used herein, the term "inhibitor of the interaction between VEGF and its target sequences, particularly its receptor(s)" is a molecule which can prevent the interaction between VEGF and its target sequences, particularly its receptor(s), particularly VEGF-R1, by competition or by fixing to one of the molecules.
Preferably, the VEGF inhibitor is an inhibitor of the interaction between VEGF and its receptor, preferably VEGF-R1, VEGF-R2 or VEGF-R3. Preferably the receptor is VEGF-R1. Preferably, it is a chemical molecule, a peptide, a protein or an antibody.
By "peptide", it is meant an amino acid sequence comprising from 2 to 30 amino acids. By "protein", it is meant an amino acid sequence comprising at least 31 amino acids, preferably 50 to 500 amino acids. By "antibody", it is meant a substance composed of four polypeptide chains, namely two light chains and two heavy chains.
Preferably, the VEGF inhibitor is an antibody, and preferably bevacizumab, ranibizumab or ramucirumab.

Preferably, the VEGF inhibitor is a molecule which can prevent the activation of VEGFR. Such a VEGF inhibitor is called a VEGFR tyrosine kinase inhibitor.
The test for determining whether a compound is a VEGFR tyrosine kinase inhibitor is the VEGFR kinase assay, and notably the VEGF-R2 kinase assay:
Kinase reaction is performed in a 10 µL volume in low-volume 384-well plates, NBS Corning model 4514 (black); the concentration of substrate in the assay is 200 nM, and the kinase reaction buffer consists of 50 mM HEPES pH 7.5, 0.01% BRIJ-35, 10 mM MgCl₂, and 1 mM EGTA. Kinase reactions (in the presence and in the absence of compounds) start by adding 30 ng/mL VEGF-R2, and are allowed to proceed for 1 hour at room temperature before a 10 µL preparation of EDTA (20 mM) and Tb-labeled antibody (4 nM) in TR-FRET dilution buffer are added. The final concentration of antibody in the assay well is 2 nM, and the final concentration of EDTA is 10 mM. The plate is allowed to incubate at room temperature for 60 minutes before being read on a plate reader configured for LanthaScreen™ TR-FRET. VEGF-R1 and VEGF-R3 kinase assays would be run in a similar fashion.

Preferably, the VEGFR tyrosine kinase inhibitor is chosen from:
Sorafenib (4-[4-[[4-chloro-3-(trifluoromethyl)phenyl]carbamoylamino] phenoxy]-N-methyl-pyridine-2-carboxamide)
Sunitinib (N-(2-diethylaminoethyl)-5-[(Z)-(5-fluoro-2-oxo-1 H-indol-3-ylidene)methyl]-2,4-dimethyl-1 H-pyrrole-3-carboxamide)
Pazopanib (5-({4-[(2,3-Dimethyl-2H-indazol-6-yl)methylamino]pyrimidin-2-yl}amino)-2-methylbenzenesulfonamide)
Vandetanib (N-(4-bromo-2-fluorophenyl)-6-methoxy-7-[(1-methylpiperidin-4-yl)methoxy]quinazolin-4-amine)
Axitinib (N-Methyl-2-[[3-[(E)-2-pyridin-2-ylethenyl]-1 H-indazol-6-yl]sulfanyl]benzamide)
Cediranib (4-[(4-fluoro-2-methyl-1H-indol-5-yl)oxy]-6-methoxy-7-[3-(pyrrolidin-1-yl)propoxy]quinazoline)
Cabozantinib (N-(4-((6,7-Dimethoxyquinolin-4-yl)oxy)phenyl)-N'-(4-fluorophenyl)cyclopropane-1,1-dicarboxamide)
Foretinib (N-(3-fluoro-4-((6-methoxy-7-(3-morpholinopropoxy)quinolin-4-yl)oxy)phenyl)-N-(4-fluorophenyl)cyclopropane-1,1-dicarboxamide)
Tivozanib (1-{2-Chloro-4-[(6,7-dimethoxyquinolin-4-yl)oxy]phenyl}-3-(5-methylisoxazol-3-yl)urea)
Lenvatinib (4-[3-Chloro-4-(cyclopropylcarbamoylamino)phenoxy]-7-methoxy-quinoline-6-carboxamide)
Lucitanib (6-({7-[(1-Aminocyclopropyl)methoxy]-6-methoxy-4-quinolinyl}oxy)-N-methyl-1-naphthamide)
Dovitinib (4-amino-5-fluoro-3-(5-(4-methylpiperazin-1-yl)-1H-benzo[d]imidazol-2-yl)quinolin-2(1 H)-one)
Orantinib ((Z)-3-(2,4-dimethyl-5-((2-oxoindolin-3-ylidene)methyl)-1H-pyrrol-3-yl)propanoic acid)
Nintedanib (Methyl (3Z)-3-{[(4-{methyl[(4-methylpiperazin-1-yl)acetyl]amino}phenyl)amino](phenyl)methylidene}-2-oxo-2,3-dihydro-1H-indole-6-carboxylate)
Vatalanib (N-(4-chlorophenyl)-4-(pyridin-4-ylmethyl)phthalazin-1-amine)
Telatinib (4-(((4-((4-chlorophenyl)amino)furo[2,3-d]pyridazin-7-yl)oxy)methyl)-N-methylpicolinamide)
Motesanib (N-(3,3-Dimethyl-2,3-dihydro-1 H-indol-6-yl)-2-[(pyridin-4-ylmethyl)amino]pyridine-3-carboxamide)
OSI-930 (3-((quinolin-4-ylmethyl)amino)-N-(4-(trifluoromethoxy)phenyl)thiophene-2-carboxamide)
Brivanib alaninate ((R)-(S)-1-((4-((4-fluoro-2-methyl-1H-indol-5-yl)oxy)-5-methylpyrrolo[2,1-f][1,2,4]triazin-6-yl)oxy)propan-2-yl 2-aminopropanoate)
BMS-690514 ((3R,4R)-4-amino-1-((4-((3-methoxyphenyl)amino)pyrrolo[2,1-f][1,2,4]triazin-5-yl)methyl)piperidin-3-ol)
Linifanib (1-[4-(3-amino-1H-indazol-4-yl)phenyl]-3-(2-fluoro-5-methylphenyl)urea) AEE788 (6-[4-[(4-Ethylpiperazin-1-yl)methyl]phenyl]-N-[(1R)-1-phenylethyl]-7H-pyrrolo[2,3-d]pyrimidin-4-amine)
TAK-593 (N-(5-((2-(cyclopropanecarboxamido)imidazo[1,2-b]pyridazin-6-yl)oxy)-2-methylphenyl)-1,3-dimethyl-1 H-pyrazole-5-carboxamide)
CEP-11981 (13-isobutyl-4-methyl-10-(pyrimidin-2-ylamino)-1,2,4,7,8,13-hexahydro-6H-indazolo[5,4-a]pyrrolo[3,4-c]carbazol-6-one)
CP-547,632 (3-((4-bromo-2,6-difluorobenzyl)oxy)-5-(3-(4-(pyrrolidin-1-yl)butyl)ureido)isothiazole-4-carboxamide), and
Regorafenib (4-[4-({[4-Chloro-3-(trifluoromethyl)phenyl]carbamoyl}amino)-3-fluorophenoxy]-N-methylpyridine-2-carboxamide).

The VEGF inhibitor is advantageously formulated in a pharmaceutical composition, together with a pharmaceutically acceptable carrier.
"Pharmaceutically" or "pharmaceutically acceptable" refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.
The form of the pharmaceutical compositions, the route of administration, the dosage and the regimen naturally depend upon the condition to be treated, the severity of the illness, the age, weight, and sex of the patient, etc.
The pharmaceutical compositions of the invention can be formulated for a topical, oral, intraocular, intravenous, intramuscular or subcutaneous administration and the like. Preferably they are topical.

Preferably, the pharmaceutical compositions contain vehicles which are pharmaceutically acceptable for a formulation capable of being topically applied.
By topical route, the composition, in particular the pharmaceutical composition, according to the invention, can be present in all galenic forms normally used for topical administration. By way of non-limiting example of topical preparations, preparations may be mentioned in liquid, pasty or solid form and, more particularly, in the form of ointments, aqueous, aqueous-alcoholic or oily solutions, dispersions of the optionally two-phase lotion type, serum, aqueous, anhydrous or lipophilic gels, powders, soaked pads, syndets, wipes, sprays, foams, sticks, shampoos, compresses, washing bases, emulsions of liquid or semi-liquid consistency such as milk, obtained by dispersing a fatty phase in an aqueous phase (O/W) or inversely (W/O), a microemulsion, suspensions or emulsions of soft, semi-liquid or solid of white or colored cream type, gel or ointment, suspensions of microspheres or nanospheres or lipid or polymeric vesicles, or microcapsules, micro- or nanoparticles or of polymeric or gelled patches allowing a controlled release.

It is routine for those skilled in the art to adjust the nature and amount of the additional active ingredients and excipients in the pharmaceutical composition so as not to affect the desired properties thereof, notably with regard to the stability of the VEGF inhibitor according to the invention, and the route of administration considered.

The physiologically acceptable medium may comprise various excipients. By "excipient" it is meant an inert substance typically used as a diluent or carrier for the VEGF inhibitor according to the invention.
Emulsions such as oil-in-water (O/W) or water-in-oil (W/O) systems, as well as a base (vehicle or support) for the topical formulation, may be chosen so as to ensure efficacy of the active ingredients and/or to avoid allergic and irritant reactions. The compositions may comprise an emulsifier. Non-limiting examples of emulsifiers useful in this regard include glycol esters, fatty acids, fatty alcohols, fatty acid esters of glycols, fatty esters, fatty ethers, glycerine esters, propylene glycol esters, polyethylene glycol fatty acid esters, fatty acid esters of polypropylene glycol, sorbitol esters, esters of sorbitan anhydrides, copolymers of carboxylic acids, glucose esters and ethers, ethoxylated ethers, ethoxylated alcohols, alkyl phosphates, polyoxyethylene phosphate ethers, fatty acid amides, acyl lactylates, soaps and mixtures thereof. Specific non-limiting examples of emulsifiers useful in the present compositions include polyethylene glycol-20 sorbitan monolaurate (polysorbate-20), polyethylene glycol, soybean sterol, steareth-2, steareth-20, steareth-21, ceteareth-20, glucose methyl ether distearate PPG-2, ceteth-10, polysorbate-80, cetyl phosphate, potassium cetyl phosphate, diethanolamine cetyl phosphate, polysorbate-60, glyceryl stearate, PEG-100 stearate, tragacanth gum and mixtures thereof.
The lotions useful in the present compositions may be suspensions of powdered material in an aqueous or alcoholic base.
Ointments are oleaginous compositions that contain little or no water (anhydrous).
The compositions can also be in the form of gels. In this regard, the compositions may comprise a gelling agent and/or a thickener. Suitable gelling and/or thickening agents which may be useful in the present compositions include aqueous thickeners, such as neutral, anionic and cationic polymers, and mixtures thereof. Examples of polymers which may be useful in the present compositions include carboxyvinyl polymers, such as carboxypolymethylene. A preferred thickener is a carbomer, for example a Carbopol® polymer from Noveon Inc. Other examples of polymers useful in this regard include hydrophilic/hydrophobic graft copolymers, such as polymers formed as a mixture of polystyrene/microsponge/Carbopol®. Such a polymer in this respect is a dimethyl acrylamide/acrylic acid/polystyrene ethyl methacrylate copolymer, for example a copolymer of the Pharmadur® brand as available from Polytherapeutics.
Other non-limiting examples of suitable thickeners include cellulosic polymers such as arabic gum, tragacanth gum, locust bean gum, guar gum, hydroxypropylguar, xanthan gum, cellulose gum, sclerotium gum, carrageenan gum, karaya gum, cellulose, rosin, methylcellulose, hydroxyethylcellulose, hydroxypropylcellulose, hydroxymethylcellulose, hydroxypropylmethylcellulose, methylhydroxyethylcellulose, cetylhydroxyethylcellulose, carboxymethylcellulose, corn starch, hydroxypropyl phosphate starch, PEG-150/alkoxy stearyl alcohol/SMDI copolymer, PEG-180/laureth-50/TMMG copolymer, acrylic acid/acrylamidomethylpropane sulfonic acid copolymer, acrylate/C10-30 acrylate copolymer, acrylate/beheneth-25 methacrylate copolymer, acrylate/steareth-20 methacrylate copolymer, acrylate/stearth-20 copolymer, acrylate/VA copolymer, ammonium acryloyldimethyltaurate/beheneth-25 methacrylate, ammonium acryloyldimethyltaurate/VP copolymer, caprylic/capric triglyceride (and) sodium acrylate copolymer, propylene glycol alginate, dimethicone and mixtures thereof. Other thickeners and/or gelling agents, such as polyacrylic polymers, may be used.

In another embodiment, the pharmaceutical composition may be adapted for injection. The compositions may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The doses used for the administration can be adapted as a function of various parameters, and in particular as a function of the mode of administration used, of the relevant pathology, or alternatively of the desired duration of treatment. For example, it is well within the skill of the art to start doses of the compound at levels lower than those required to achieve the desired therapeutic effect and to gradually increase the dosage until the desired effect is achieved. However, the daily dosage of the products may be varied over a wide range from 0.01 to 1,000 mg per adult per day. Preferably, the compositions contain 0.01, 0.05, 0.1, 0.5, 1.0, 2.5, 5.0, 10.0, 15.0, 25.0, 50.0, 100, 250 and 500 mg of the active ingredient for the symptomatic adjustment of the dosage to the subject to be treated. A medicament typically contains from about 0.01 mg to about 500 mg of the active ingredient, preferably from 1 mg to about 100 mg of the active ingredient. An effective amount of the drug is ordinarily supplied at a dosage level from 0.0002 mg/kg to about 20 mg/kg of body weight per day, especially from about 0.001 mg/kg to 7 mg/kg of body weight per day.

According to the invention, 'treatment' includes therapeutic treatment, and 'prevention' includes prophylactic or preventive treatment, wherein the object is to prevent or slow down the targeted pathologic condition or disorder. Those in need of treatment include those already with the disorder as well as those prone to have the disorder or those in whom the disorder is to be prevented. The term "treating" includes reducing, alleviating or inhibiting or eliminating the symptoms or progress of a disorder.
Preferably, an effective amount, preferably a therapeutically effective amount of the VEGF inhibitor of the invention is administered. An "effective amount" refers to an amount effective, at dosages and for periods of time necessary, to achieve the desired therapeutic or prophylactic result.
A "therapeutically effective amount" of a VEGF inhibitor of the invention may vary according to factors such as the disease state, age, sex, and weight of the individual, and the ability of the VEGF inhibitor, to elicit a desired therapeutic result. A therapeutically effective amount encompasses an amount in which any toxic or detrimental effects of the VEGF inhibitor are outweighed by the therapeutically beneficial effects. A therapeutically effective amount also encompasses an amount sufficient to confer benefit, e.g., clinical benefit.

The invention will be better understood on reading the following illustrative and non-limiting examples.

### Example: Dermal cDC1 control sustained recruitment and survival of neutrophils through VEGFα secretion in response to cutaneous bacterial infection

### MATERIALS AND METHODS

### Mouse models

C57BL/6 (C45.2⁺) mice were from the Biological Resource Center, Agency for Science, Technology and Research (A*STAR), Singapore. Congenic C57BL/6 (C45.1⁺) mice were from Jackson Laboratory. Langerin DTR mice were provided by B. Malissen. Batf3^{-/-} and ZBTB46^{-GFP} BM were provided by K. Murphy. HIF^{flox} mice and IRF4^{flox} mice were crossed with CD11c cre⁺ mice to give rise to congenic CD11c cre⁺HIF^{flox} mice and CD11c cre⁺IRF4^{flox} mice respectively. All mice were bred and maintained in the Singapore Immunology Network animal facility before use at 7-10 weeks of age. For the generation of BM chimeras, female B6 CD45.1 mice at 6-8 weeks of age were lethally irradiated with two doses of 600 rads, 5 hr apart, and then injected i.v. with 1 x 10⁶ cells from femurs and tibias of Batf3^{-/-}, Zbtb46^{gfp}, and langerin DTR mice. All experiments and procedures were approved by the Institutional Animal Care and Use Committee of the Biological Resource Center (Agency for Science, Technology and Research, Singapore) in accordance with the guidelines of the Agri-Food and Veterinary Authority and the National Advisory Committee for Laboratory Animal Research of Singapore.

### Bacterial culture and intra-dermal injection

*P. acnes* strain ATCC 6919 was obtained from American Type Culture Collections and grown in an anaerobic chamber at 37°C for 5-6 days. A spectrophotometer reading OD₅₂₅ was used to identify the bacterial log growth phase. Live *P. acnes* was intradermally injected (10⁸ CFU in 20µl of PBS) into the left ear of mice; while the right ear was injected with an equal volume of PBS. For infection in langerin-DTR mice, DT was administered intraperitoneally (i.p.). Recombinant mouse VEGF 164 protein (300 ng, R&S system) or anti-vegfa (2µg, Abcam) have been intradermally injected concomitantly with *P. acnes.* Ear thickness was measured using an electronic caliper (Mitutoyo).

### Histological analysis

Frozen skin samples embedded in OCT compound (Sakura Finetek) were sliced into 5-µm-thick sections and fixed with 1% paraformaldehyde (PFA) before staining with Mayer's hematoxylin and eosin (H&E) or picosirius red.

### Preparation of ear cell suspensions

Mouse skin cells were isolated as described previously [Ginhoux, F., M. P. Collin, et al. (2007). "Blood-derived dermal langerin+ dendritic cells survey the skin in the steady state." J Exp Med 204(13): 3133-3146]. Briefly, mouse ears were split into dorsal and ventral halves and floated in RPMI-1640 medium (Sigma) containing 1 mg/ml dispase (Invitrogen) for 60 min at 37°C to allow separation of epidermal and dermal sheets. The separated sheets were then cut into small pieces and incubated in RPMI containing 10% serum, Collagenase IV (0.2mg / ml, Roche) and DNAse I (20000U / ml, Roche) for 1 hr at 37°C. Cells were then passed through 19 G needle and filtered through a 100 µM cell strainer (BD Falcon) to obtain a homogenous cell suspension.

### Flow cytometry and fluorescence-activated cell sorting

Multi-parameter analyses and sorting of labelled cell suspensions were performed on a LSR II and FACSAria II (Becton Dickinson, San Jose, USA) respectively. Data were analysed with FlowJo software (TreeStar). Fluorochrome- or biotin- conjugated monoclonal antibodies (mAbs) against the following molecules used: mouse IA/IE (M5/114.15.2) (BD Biosciences); Ly6G (1A8), CD64 (X54-5/7.1), EpCAM (G8.8) (Biolegend) and CD11c (N418), CD45 (30F11), CD45.1 (A20), CD45.2 (104), Ly6C (HK1.4), CD24 (M1/69), FCεRI (MAR-1), BST2 (eBio927), and CD11b (M1/70) (all from eBioscience). The streptavidin-CF594 conjugate (562284) was from BD Biosciences.

### Mass Cytometry by Time Of Flight (CyTOF)

To enable detailed characterization of the granulocytic and myeloid populations within the inflammatory infiltrate, cell suspensions from mouse ears were first depleted of T and B cells using antibodies against CD90 and CD19, respectively. The remaining cells were placed in a U-bottom 96-well plate (BD Falcon, cat. no. 3077), washed once with 200 µL flow cytometry buffer (4% FBS, 2 mM EDTA, 0.05% Azide in 1 × PBS) and then stained with 100 µL of 200 µM cisplatin (Sigma-Aldrich, cat. no. 479306-1 G) for 5 min on ice to exclude dead cells. Cells were washed twice with flow cytometry buffer and incubated with 50 µL heavy-metal isotope-labeled secondary mAb cocktail for 30 min on ice. Purified antibodies were obtained from Beckton Dickinson, BioLegend, eBioscience, BioXcell, R&D Biosystems and AbD Serotec using clones listed in **Table 1**:

**Table 1: Table of antibodies used for mass cytometry analysis listing metal conjugate, antibody clone name, supplier**

| **Isotope tag** | **Sequence** | **Clone/Company** |
|---|---|---|
| Qdot (Cd112/114) | CD19 | 6D5 (Invitrogen) |
| Rh--103 | *Barcode* | |
| Pd--104 | *Barcode* | |
| Pd---105 | *Barcode* | |
| Pd--106 | *Barcode* | |
| Pd--108 | *Barcode* | |
| Pd--110 | *Barcode* | |
| Ln--113 | *Barcode* | |
| Pr--141 | CD45 | 30--F11 (Biolegend) |
| Nd--142 | MHC class II | Y3P (BioXcell) |
| Nd--143 | B220 | RA3.341/61 (BioXCell) |
| Nd--144 | CD11a | FD441.8 (BD) |
| Nd--145 | Ly6G/C | RB6--8C5 (BD) |
| Nd--146 | CD8 | 53--6.7 (BD) |
| Sm--147 | Ly6G | IA8 (BD) |
| Nd--148 | Ly6c | HK1.4 (Biolegend) |
| Sm--149 | CD4 | H129.19 (BD) |
| Nd--150 | CD54 | YNI.7.4 (BioXCell) |
| Eu--151 | CD62L | MEL14 (Biolegend) |
| Sm--152 | CD49B | DX5 (Biolegend) |
| Eu--153 | CD11b | M1/70 (BD) |
| Sm--154 | CD3 | 145.2C11 (BD) |
| Gd--155 | CD86 | GL7 (BD) |
| Gd--156 | BST2 | eBio927 (ebioscience) |
| Gd--157 | Mertk (SAv) | Mertk--biotin-- accession number 60805 (R&D Systems) |
| Gd--158 | CD134 | ACT35 (Biolegend) |
| Tb--159 | F4/80 | C1:A3-1 (Serotec) |
| Gd--160 | FcER1 | MAR--1 (ebioscience) |
| Dy--161 | CD43 | eBioR2/60 (ebioscience) |
| Dy--162 | PDL--1 | 10F.9G2 (Biolegend) |
| Dy--163 | CD11c | N418 (Biolegend) |
| Dy--164 | CD103 | 2e7 (Biolegend) |
| Ho--165 | CD64 | X54--5/7.1 (Biolegend) |
| Er--166 | CD48 | HM48--1 (Biolegend) |
| Er--167 | CCR2 (a--APC) | CCR2 APC -- 475301 (R&D systems) |
| | | a--APC -- APC003 (Biolegend) |
| Er--168 | CD44 | IM7 (Biolegend) |
| Tm--169 | CD115 | AF5 (purified in--house) |
| Er--170 | CD38 | 90 (Biolegend) |
| Yb--171 | SiglecH | 551 (Biolegend) |
| Yb--172 | CD301 (a--PE) | CD301 PE -- LOM--14 (ebioscience) |
| | | aPE -- PE001 (Biolegend) |
| Yb--173 | SIRPa (CD172a) | P84 (BD) |
| Yb--174 | CD24 | M1/69 (Biolegend) |
| Lu--175 | ESAM | 1 G8 (ebioscience) |
| Yb--176 | CD90 | T24/31 (BioXcell) |
| Ir--191/193 | DNA | DVS Sciences |
| 195 | CisPlatin | Sigma Aldrich |
| | *live*/*dead* | |
| Cell length | *Cell length* | N/A |

For some markers fluorophore-(phycoerythrin or allophycocyanin) or biotin-conjugated antibodies were used as primary antibodies followed by secondary staining with anti-fluorophore metal-conjugated antibodies (anti-APC, (Clone APC003, BioLegend cat. no. 408002, 5 µg/mL), anti-PE, (Clone PE001, BioLegend cat. no. 408102, 5 µg/mL)) or metal-conjugated streptavidin. Cells were washed twice with flow cytometry buffer then once with PBS before fixing with 200 µL 2% PFA (Electron Microscopy Sciences, cat. no. 15710) in PBS overnight. After fixation, cells were pelleted and resuspended in 200 µL 1 × perm buffer (BioLegend, cat. no. 421002). Cells were washed once with perm buffer and then PBS before barcoding. In order to reduce technical variation, the inventors used mass-tag barcoding which permits acquisition of data from all cell suspensions from all the time points and tissues during a single analysis. A unique combination of the following barcodes was chosen to stain each tissue sample for 30 min on ice: DM-Rh-103, BABE-Pd-104, BABE-Pd-105, BABE-Pd-106, BABE-Pd-108, BABE-Pd-110 and BABE-Pd-113. Cells were next washed with perm buffer, incubated with CyFACS for 5 min on ice, washed once more with CyFACS, and labeled at room temperature with 250 nM iridium interchalater (DVS Sciences) diluted in PBS with 2% PFA. After 20 min, cells were washed twice with CyFACS and twice in distilled water before diluting to 0.5 x 10⁶ cells/ml in water. Cell data were acquired and analyzed using a CyTOF Mass cytometer. The data were exported in a traditional flow cytometry file (.fcs) format and cells for each barcode were deconvoluted using Boolean gating using FlowJo software (Tree Star, Ashland, USA). tSNE and One-SENSE analyses were performed as recently described [Cheng, Y., M. T. Wong, et al. (2016). "Categorical Analysis of Human T Cell Heterogeneity with One-Dimensional Soli-Expression by Nonlinear Stochastic Embedding." J Immunol 196(2): 924-932; and Guilliams, M., C. A. Dutertre, et al. (2016). "Unsupervised High-Dimensional Analysis Aligns Dendritic Cells across Tissues and Species." Immunity 45(3): 669-684].

### Western blotting

After isolation of skin-infiltrating neutrophils by cell sorting, cells were snap frozen and homogenized in Histone extraction buffer (Abcam) supplemented with protease inhibitor cocktails (Roche) according to the manufacturer's protocol. The protein content of the supernatant was determined by bicinchoninic acid protein assay; an equal amount of protein per sample was then resolved on gradient gels (4-12% Bis-Tris Plus gels, Life Technologies) and transferred onto PVDF membranes. Membranes were incubated with primary antibodies (rabbit polyclonal anti-H3Cit, 1:1,000, Abcam, cat. no. ab5103; rabbit polyclonal anti-H3, 1:1,000, Abcam, cat. no. ab1791, goat monoclonal anti-MMP9, 1:800, R&D, cat. no. AF909, mouse monoclonal anti-Actin, 1:5,000, Abcam, cat. no. ab3280) at 4°C overnight, and subsequently with appropriate HRP-conjugated secondary antibodies. Specific staining was visualised using enhanced chemiluminescence substrate (GE Healthcare). Equal loading was confirmed by probing for Actin or Histone. Band intensity was measured using Image Lab software.

### Microarray analysis of gene expression

RNA was extracted from mouse skin samples using a mirVana miRNA isolation kit (Ambion), and was prepared for microarray (Illumina Mouse WG6) according to the manufacturer's instructions (Illumina). Microarray data were logarithmically transformed (i.e log2) for variance stabilization, followed by quantile normalization. Limma was used to select genes that were up- or down- regulated in *P. acnes* injected mice at days 2 and 6 post-injection, individually, by using D0 as the control group. Differentially-expressed genes (DEG) were selected based on a Benjamin-Hochberg adjusted p-value lower than 0.05, and with a minimum log-fold-change of 1.5.

### Single-cell capture and library preparation for RNA sequencing

Skin cDC1 were isolated after *P. acnes* infection by fluorescence-activated cell sorting, and were diluted to a final concentration of 400 cells per µl. Cells were loaded onto C₁ integrated fluidic circuits (IFC) (5- to 10-µm chip) for capture, cell lysis, reverse transcription with oligo (dT) primers, and amplification of cDNA on a C1 Single-cell Auto Prep System according to the protocol of the manufacturer (Fluidigm). Reads were mapped against the mm10 reference genome with the Bowtie alignment program. The estimated expression values, in transcripts per million (TPM), for each gene annotated with gencode gene annotation version M4 was calculated using the RSEM program. Genes with a TPM value of 0 in all cells were discarded. TPM values less than 1 were considered background, and log2 TPM was set to 0 as a conservative background cut-off. Positive TPM values then underwent log2 transformation. There were no outlier cells identified with the Singular Analysis Toolset with default parameters. Using the Singular Analysis Toolset, the inventors selected a subset of 8238 genes whose log2 TPM values were greater than 1 at both sample-average level and across all the cells, for all downstream analyses. The PCA function, using genes expressed in more than two cells with a non-zero variance, highlighted and ranked the genes with the most variability in expression across all the cells (data not shown); together with the hierarchical clustering function in the Singular Toolset (data not shown) this identified two subsets comprising 8 and 59 cells respectively. The ANOVA function in the Singular Toolset identified genes that were significantly differentially expressed in these two cell subsets: 1796 genes were identified, with *P* values of < 0.05. Among these genes, 1556 were upregulated and 240 were downregulated in the cell subset comprising of 8 cells, compared to the larger 59-cell subset. Multiple-testing correction was done by the Benjamini-Hochberg method. The sorting points into neighborhoods (SPIN) algorithm was applied to the top 100 genes with highest loadings associated with the first principal component of PCA and independently identified the two cell subsets comprising the same individual cells (data not shown). Pathway enrichment analysis was performed using the IPA software on the 240 genes expressed more abundantly in the minor subset, with *P* values < 0.05 considered significant. Except for the Matlab implementation of SPIN, or where otherwise noted, all analyses were performed with software of the R project for statistical computing, version 3.3.0 (Bioconductor).

### Bone marrow-derived DC culture

Bone marrow was flushed from the femur and tibia from C57BL/6 mice and was used without any digestion. BM cells were incubated in 10 mL of RPMI 1640 medium with 10% heat-inactivated fetal calf serum (Biochrom, Cambridge, UK), penicillin/streptomycin, and 50 mM β-mercaptoethanol with FLT3L (200 ng/ml) combined with GM-CSF (5 ng/ml) for 9 days. Cells were then replated with the same combination of cytokines, and collected at day 15.

### Gene expression analysis using NanoString nCounter system

A total of 179 immunology-related mouse genes and six internal reference genes (**Table 3**) were implemented in the digital transcript counting (nCounter GX Mouse Inflammation Kit NanoString, Seattle, WA).

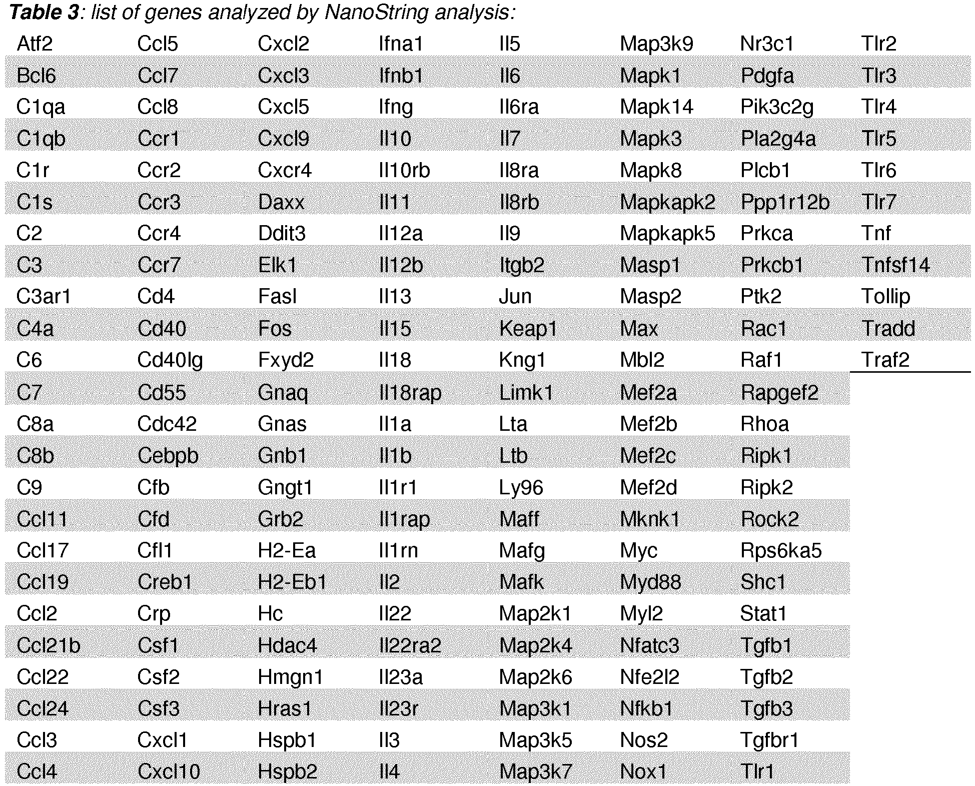

Total RNA (100 ng) was assayed on a nCounter Digital Analyzer (NanoString) according to the manufacturer's instructions. Data were normalized by scaling with the geometric mean of the built-in control gene probes for each sample. Then, a log transformation (base 2) was performed. The heat maps showing levels of gene expression for differentially-expressed genes between neutrophils infiltrating the skin in the absence or presence of cDC1 were plotted and analysed using the GENE-E software, version 3.0.230 (Broad Institute, Cambridge, MA)

### Intravital multiphoton imaging

The inventors used Lysozyme M-GFP/ langerin-DTR albino mice for all intravital imaging experiments, wherein all the cells expressing lysozyme (neutrophils) are labeled green [Li, J. L., C. C. Goh, et al. (2012). "Intravital multiphoton imaging of immune responses in the mouse ear skin." Nat Protoc 7(2): 221-234; and Scheiermann, C., P. S. Frenette, et al. (2015). "Regulation of leucocyte homeostasis in the circulation." Cardiovasc Res 107(3): 340-351]. Imaging of the ear was performed as previously described (Li at al 2012). In brief, mice were anaesthetized with a cocktail of ketamine and xylazine (10 µl per gr of body weight) and then placed onto a heat pad to maintain body temperature at 37°C throughout the imaging procedure. Depilatory cream was applied for 2 min to remove hair on the upper two-thirds of the dorsal side of the mouse ear. The ear was then mounted on a custom-made ear skin stage for intravital multiphoton imaging. Data correction and analysis were conducted using Imaris software (Bitplane, South Windsor, CT). Multiphoton mosaic imaging was performed to capture the entire volume of the granuloma and its surrounding interstitium. The granuloma was quantified on Imaris using the surfacing tool, with a voxel size of 1 um. The percentage of granuloma was calculated (total volume of granuloma/total imaged volume *100; where total imaged volume was normalized across experiments).

### RESULTS

### Intra-dermal injection of P. acnes causes ear swelling, successive waves of immune cell infiltration, and fibrosis

To characterize the longitudinal immune response to *P. acnes* infection, the inventors injected 10⁸ colony forming units (CFU) of live *P. acnes* into the dermis of the left ears of wild-type C57BL/6 (WT B6) mice, with PBS injected into the dermis of the right ears as a control, and measured the resulting swelling at intervals over 75 days. Ear thickness was significantly greater in *P*. acnes-injected ears compared to PBS-injected ears from 8hrs post-injection (*P. acnes* versus PBS control: 0.455 ± 0.024 mm vs 0.236 ± 0.018 mm; p=0.0022) and throughout the experiment (*P. acnes* D75 versus PBS control: 0.29 ± 0.03 mm vs 0.205 ± 0.018 mm; p=0.0119) (figure not shown). The greatest difference in ear thickness was observed on day 9 (*P. acnes* versus PBS control: 0.8 ± 0.08 mm vs 0.216 ± 0.017 mm; p=0.0004), then slowly declined (figure not shown). The inventors observed increased opacity around the *P. acnes* injection site from day 4 post-injection, consistent with a granulomatous reaction (Boros, 2013), accompanied by marked cellular infiltration (figure not shown). Interestingly, sections from ears on day 70 exhibited increased staining for collagen fibers (figure not shown), consistent with abnormal skin reorganization and fibrosis (Lattouf et al., 2014).

To define the composition of the immune cell infiltrate during the acute phase of inflammation following *P. acnes* infection, the inventors performed a comprehensive high-dimensional analysis of the ear cell populations using mass Cytometry by Time Of Flight (CyTOF) (Spitzer and Nolan, 2016, Ornatsky et al., 2006, Bendall et al., 2011, Tanner et al., 2013, Becher et al., 2014). Cell suspensions were generated (Ginhoux et al., 2007) from *P. acnes-* and PBS- injected ears at 12 hours, and 2 and 5 days post-injection. The inventors then labeled the cells with a panel of antibodies recognizing 37 different molecules (**Table 1**) to allow the inventors to identify the different myeloid and granulocytic populations present (figure not shown). The CyTOF data were analyzed using One-SENSE (One-dimensional Soli-Expression by Nonlinear Stochastic Embedding) (Cheng et al., 2015), a new dimensionality-reduction method based on t-stochastic neighbor embedding (tSNE), which enables unbiased visualization of high-dimensional similarities of cells in a two-dimensional map. One-SENSE allows to first define expression of lineage-imprinted markers as the first dimension, thereby grouping broadly-ontogenetically-related cells together, and then to allow the other markers to define the second dimension, thus revealing any heterogeneity within the main clusters (figure not shown), as recently published for the analysis of murine tissue DC composition (Guilliams et al., 2016). The inventors defined the "lineage dimension" using 5 markers that are differentially-expressed by immune cell populations: Ly6G for neutrophils, Ly6C for monocytes, CD11b and MHC-II for DC, and CD64 for macrophages (figure not shown). The association of this "lineage dimension" with the "marker dimension" resulted in the unbiased generation of multiple clusters of cells of different lineages, allowing to visualize all granulocytic and myeloid cell subsets infiltrating the skin over the first 5 days of the immune response to *P. acnes* (figure not shown).

The CyTOF data revealed within the Ly6G⁻ cell population (figure not shown), the presence of LC (CD11b^{int}MHCII^{hi}CD64^{neg}CD24^{hi}), cDC1 (CD11b^{neg}MHCII^{hi}CD64^{neg}CD24^{hi}), cDC2 (CD11b^{hi}MHCII^{hi}CD64^{neg}CD172a^{hi}), CD11b⁻CD103⁻ double negative (DN) DC (CD11b^{neg}MHCII^{hi}CD24^{neg}CD64^{neg}), mast cells (CD11b^{neg}MHCII^{neg}CD64^{neg}FCεRI^{int}), Ly6C^{lo} monocytes (CD11b^{hi}MHCII^{neg}CD64^{neg}Ly6C^{neg/low}), Ly6C^{hi} monocytes (CD11b^{hi}MHCII^{neg}CD64^{neg}Ly6c^{hi}CCR2^{hi}) and macrophages (CD11b^{hi}MHCII^{neg}CD64^{hi}Ly6C^{neg}). The remaining CD11b^{hi}MHCII^{hi}CD64^{int-hi}Ly6C^{low}FCεRI^{lo-int} cells were classified as monocyte-derived inflammatory cells (MC), with phenotypic features of both macrophages and DC (Guilliams et al., 2014, Ginhoux et al., 2012, Malissen et al., 2014), but distinguishable by their expression of CD64 and low-intermediate levels of Ly6C (figure not shown), coupled with lack of expression of the DC lineage marker, zinc finger transcription factor Zbtb46 (Btbd4) (figure not shown) (Satpathy et al., 2012a, Satpathy et al., 2012b, Meredith et al., 2012).

The inventors then confirmed the presence and absolute abundance of these populations in skin cell suspensions during the first 30 days post-injection with *P. acnes* using conventional flow cytometry (figure not shown). From these data the inventors can see that four distinct waves of immune cells pass through *P*. *acnes*-injected skin: from 8 hours, neutrophils, which peak around the end of day 1 and decline to disappearance around day 9; then Ly-6C^{high} monocytes which although peaking at day 1 accumulate at high level from day 4 to day 9 declining between days 9 and 15 post-infection; while MC accumulate from day 5, peaking at day 9, consistent with their likely derivation from the earlier-infiltrating monocytes and finally macrophages accumulating slowly overtime until day 9 (figure not shown); lastly, T lymphocytes infiltrate the skin from day 5 and remain abundant until day 15 (figure not shown).

### cDC1 are required for sustained immune cell recruitment following intra-dermal injection of P. acnes

DC control the hepatic granulomatous reaction to heat-killed *P. acnes* in mice (Ohteki et al., 2006); the inventors therefore asked what role these cells play in the immune response to dermal infection with *P. acnes.* In Langerin-DTR mice, exposure to diphtheria toxin (DT) induces specific depletion of dermal cDC1 and LC (Ginhoux et al., 2007, Kissenpfennig et al., 2005). The inventors injected these mice intra-peritoneally with DT and confirmed rapid and lasting LC depletion, coupled with significant reduction in the abundance of cDC1 in the skin from 18 hours post-injection (data not shown). Ear skin swelling in response to *P. acnes* injection was similar in WT and DC-depleted Langerin-DTR mice (injected 24h post-DT) during the first day of infection (D1 *P. acnes* WT B6 mice versus D1 *P. acnes* Langerin DTR mice 24h post-DT-injection: 0.498 ± 0.043 mm vs 0.465 ± 0.024 mm; p=0.2641) (data not shown). However, from D2, the mice lacking both cDC1 and LC (LG-DTR mice 24h post-DT) exhibited significantly less ear swelling than did WT B6 mice (D9 *P. acnes* WT B6 mice versus D9 *P. acnes* Langerin DTR mice 24h post-DT-injection: 0.8 ± 0.08 mm vs 0.373 ± 0.02 mm; p=0.0.0022) (data not shown).

Thus LC and/or cDC1 seem to be required for the progressive inflammatory response to intra-dermal *P. acnes* injection.

The inventors then asked how the depletion of cDC1 and LC affected the abundance of other immune cell types in the ears of P. acnes-injected mice. The inventors observed similar numbers of neutrophils, macrophages and Ly-6C^{high} monocytes in the first 18 hours after *P. acnes* infection in the presence or absence of both cDC1 and LC (data not shown). However, from 24 hours after *P. acnes* injection the absence of langerin⁺ cells resulted in significant decreases in the number of neutrophils, macrophages and Ly-6C^{high} monocytes compared to WT mice (data not shown). This effect was not due to a defect in neutrophil generation or regress from the bone marrow, as similar absolute numbers of neutrophils were present in the bone marrow and blood of DT-treated and untreated langerin DTR mice, and in WT mice (data not shown). Together these results suggest that the early recruitment of immune cells into the dermis following *P. acnes* injection is independent of Langerin⁺ cells, but from day 1, Langerin⁺ cells are necessary for sustained neutrophil and Ly-6C^{high} monocyte infiltration.

To distinguish the roles of LC and cDC1 in this phenomenon, the inventors exploited the differential repopulation characteristics of the two cell populations in langerin-DTR mice. The inventors compared mice injected with DT 24 hours before *P. acnes* challenge, depleted of both LC and cDC1, to mice injected with DT 30 days before, in which LC remain absent while cCD1 have fully repopulated the dermis (Ginhoux et al., 2007) (data not shown). In the presence of cDC1 alone, inflammatory ear thickening in response to *P*. *acnes* injection was comparable to WT mice, again peaking around day 9 (D9 *P. acnes* WT B6 mice versus D9 *P. acnes* Langerin DTR mice (30 days): 0.8 ± 0.08 mm vs 0.72 ± 0.036 mm; p=0.0541); similar numbers of neutrophils, macrophages and Ly-6C^{high} monocytes were also present in ear cell suspensions from langerin-DTR mice 30 days after DT injection compared to WT B6 mice at all the different time points tested (data not shown). Interestingly, in the first 18 hours post *P. acnes* injection, neutrophil infiltration in the absence of LC was significantly higher than in WT mice, in line with the reported inhibitory role of LC on leucocyte infiltration (Terhorst et al., 2015). Furthermore, adoptive transfer of bone marrow-derived DC1 into Langerin DTR mice lacking LC and cDC1 (24 hours post-DT-injection), at the time of *P. acnes* injection, reinstated the inflammatory ear swelling and neutrophil infiltration up to WT levels (data not shown). Together these data show that cDC1 are both necessary and sufficient for the immune response to cutaneous *P. acnes* infection in mice.

To confirm the critical role of cDC1 in this early inflammatory response to *P. acnes,* the inventors used *Batf3*^{*-*/*-*} mice, which exhibit severe defects in the development of cDC1, but retain LC (Hildner et al., 2008) (data not shown). Following intra-dermal *P. acnes* injection, *Batf3*^{*-*/*-*} mice exhibited a similar inhibition of progressive ear swelling (data not shown) and reduced levels of immune cell infiltration (data not shown) as did LG-DTR mice depleted of dermal LC and cDC1 (24 hours post-DT injection). Thus mice lacking only cDC1 are unable to mount full and sustained inflammatory responses to *P. acnes* in the skin, supporting the key role for this DC subset during *P. acnes* infection. As LC radioresistance constitutes the best way to distinguish them from radiosensitive dermal cDC (Ginhoux et al., 2007, Merad et al., 2002), the inventors generated CD45.1 Langerin-DTR BM into CD45.2 WT B6 chimeras. In this model, DT administration will only deplete cDC1 and not LC (data not shown) and consistent with our previous observation, absence of cDC1 dampened *P. acnes* induced inflammation measured by a decrease of the ear inflammation and cell infiltration similar to the langerin-DTR mice (data not shown). Finally, the inventors asked about any potential role of cDC2 using the CD11c-cre IRF4flox model, which lacks functional cDC2 in the skin (Schlitzer et al., 2013, Bajana et al., 2012). The absence of cDC2 had no effect on *P.* acnes-induced inflammation measured by ear swelling and cell infiltration (data not shown).

Altogether these data identified a crucial role played by cDC1 in the induction of the inflammatory immune responses to *P. acnes,* in particular in the regulation of neutrophil recruitment.

### Depletion of cDC1 reduces neutrophil expression of recruitment-related genes

To begin to understand how the absence of cDC1 leads to lower neutrophil numbers during *P. acnes* infection, the inventors compared the gene expression profiles of neutrophils from the skin of non-depleted LG-DTR mice (control mice) and LG-DTR mice 24 hours post-DT-injection (depleted mice). The inventors identified 297 (178 down-regulated and 119 up-regulated), 149 (78 down-regulated and 71 up-regulated), and 227 (103 down-regulated and 124 up-regulated) differentially-expressed genes (DEG) between neutrophils from control and cDC1-depleted mice at 12 hours, and 2 and 4 days post-infection, respectively (**Table 2**). Ingenuity Pathway Analysis (IPA) revealed that most of the GO biological processes associated with the down-regulated DEGs were related to immune function of neutrophils, and specifically to neutrophil recruitment to the site of inflammation: these included cellular movement, recruitment, infiltration, quantity, chemotaxis, priming, mobilization, migration and attraction of neutrophils (data not shown). The inventors further validated these results using the quantitative nanostring platform, focusing on the expression of 179 inflammatory genes (**Table 3**). The inventors identified 28 neutrophil recruitment and migration-associated genes whose expression significantly differed in the presence or absence of cDC1 during *P. acnes* infection, including *Cdc42* (Kumar et al., 2012), *CXCL3* (Stock et al., 2014), *CCR1* (Reichel et al., 2006), *IL*-*1β* and *TNFα*. Other DEGs related to neutrophil activation/response to bacterial products were *Oas1a, IRF5* and *IRF7* (Ericson et al., 2014). Seven of these DEGs from the inflammatory gene panel overlapped with those highlighted by the bulk RNAseq analysis above (**Table 4**).
***Table 4:** overlapping DEG between neutrophil analyzed by Nanostring and bulk RNA seq* Common genes between Nanostring and Bulk RNA seq analysis

```
               CXCL10
                 IL1a
                IL23a
                CSF3
               STAT1
                IRF1
                IFIT2
```

Similarly, IPA showed that most of the GO biological processes that the DEGs were enriched for were related to neutrophil immune function, and specifically to their recruitment to inflamed sites (data not shown). Some of the GO biological processes were also related to monocyte recruitment, such as Colony Stimulating Factor 1 (CSF-1) (data not shown). Down-regulation of neutrophil CSF-1 expression likely explains the decreased monocyte recruitment observed in the absence of cDC1, as neutrophil-derived CSF-1 controls monocyte influx into inflamed tissue (Wang et al., 2016).

The inventors next used intravital multiphoton microscopy to ask whether these changes in neutrophil gene expression in the absence of cDC1 had functional correlates *in vivo* during the early stages of *P. acnes* infection. To enable visualization of neutrophils, the inventors employed albino Lysozyme M-GFP/Langerin-DTR mice, in which all cells expressing lysozyme (neutrophils) are labeled in green (Li et al., 2012) and Langerin-expressing LC and cDC can be depleted by DT administration. In non-depleted control mice, one day after *P. acnes* injection, neutrophils swarmed in a coordinated fashion, moving rapidly in the extravascular space and accumulating around the *P. acnes* injection site (data not shown). This high velocity movement remained on day 2 after *P. acnes* injection (data not shown). In LC- and cDC1- depleted mice, neutrophil density was markedly lower around the *P. acnes* infection site from day 1 post-injection (data not shown), and neutrophil velocity was significantly decreased to almost complete stillness by day 2 (data not shown). Also on day 2, there was a further decrease in the number of infiltrating neutrophils, concurrent with significantly smaller granuloma development (data not shown). In the absence of cDC1, neutrophil displacement was also significantly lower compared to non-depleted mice (data not shown). Interestingly, neutrophils were almost sessile at D2 in mice depleted of cDC1 (data not shown).

In summary, in the absence of LC and dermal cDC1, patterns of neutrophil gene expression are significantly altered, resulting in decreased expression of migration- and recruitment- associated genes, which is mirrored at the functional level by less neutrophil recruitment to the *P. acnes* injection site and decreased motility of those neutrophils that are recruited. These data support the crucial role of cDC1 in regulating the sustained and dynamic recruitment of neutrophils from the blood to the skin.

### cDC1 control essential neutrophil activation

Alongside reduced expression of migration- and recruitment- associated genes in neutrophils from mice depleted of dermal cDC1, transcripts encoding key components of anti-microbial pathways were also significantly less abundant in neutrophils in the absence of cDC1 (data not shown). One such molecule is Receptor-Interacting Protein Kinase 1 (RIPK1), which, together with RIPK3 and Mixed Lineage Kinase domain-Like (MLKL), is necessary for the release of neutrophil extracellular traps (NETs) (Desai et al., 2016) - anti-microbial webs of DNA, coated with cytotoxic histones and microbicidal proteases (Brinkmann et al., 2012). Hence, the inventors asked whether depleting cDC1 compromised the ability of neutrophils to generate NETs. A prerequisite for NETosis is the modification of arginine residues to citrulline on histones by a specific enzyme (peptidyl arginine deiminase 4, PAD4), which leads to massive chromatin de-condensation (Wang et al., 2009). Quantification of the ratio of histone H3 citrullinated (H3Cit) to normal histone H3 (H3) by Western blot revealed a significant increase in the H3Cit/H3 ratio in undepleted versus depleted mice. These data suggested that in the absence of cDC1, NETosis activity was compromised (data not shown). Another hallmark of neutrophil activation is the upregulation of Matrix Metallopeptidase 9 (MMP9) activity (Jang et al., 2015, Christoffersson et al., 2012, Bradley et al., 2012). Hence, the inventors investigated if the absence of cDC1 could impact neutrophil MMP9 expression. Although the inventors did not detected any change in MMP9 mRNA expression by RNAseq (not shown), MMP9 protein expression increased nearly two-fold between D1 and D4 after *P. acnes* infection in recruited neutrophils from the undepleted mice. However, in the absence of cDC1, MMP9 protein expression was reduced as soon as D1 post-infection and did not increase overtime (data not shown). Supporting an important role of MMP9 activity in neutrophils, MMP9 KO mice (Vu et al., 1998) exhibited reduced ear thickness and a marked impairment of neutrophil, macrophage and Ly-6C^{high} monocyte recruitment after *P. acnes* infection (data not shown). The inventors also identified a down-regulation of MMP2 expression at the gene expression level in absence of cDC1 (data not shown), a Matrix Metallopeptidase known to promote early neutrophil recruitment in association with MMP9 (Song et al., 2015). Collectively, these data demonstrate that cDC1 regulate full neutrophil activation in response to *P. acnes* infection.

### Depletion of cDC1 affects neutrophil survival

Neutrophils are short-lived cells in the steady state, but during inflammation their lifespan increases through activation-induced inhibition of apoptosis (Costantini et al., 2010, Elbim et al., 2009). Thus the reduced expression of activation-related genes in neutrophils from LC and cDC1-depleted mice might also increase neutrophil apoptosis. The inventors measured expression of the early apoptotic marker Annexin-V, and staining with the DNA dye DAPI (indicating dead cells) across the neutrophil populations from the ear skin of mice injected with *P. acnes.* From day 1 post-infection, the relative numbers of dying (Annexin-V⁺/DAPI-) and dead neutrophils (Annexin-V⁺/DAPI⁺ and Annexin-V⁻/DAPI⁺) were significantly increased in the absence of the LC and cDC1, compared to non-depleted control mice (26.37 ± 5.2% vs 15.28 ± 1.81%; p=0.0087) (data not shown). In agreement with these observations, the inventors observed an upregulation of the gene expression of *bim* (BCL2L11), a pro-apoptotic member of the Bcl-2 family (Aguilo et al., 2014), in absence of cDC1 (**Table 2**).

**Table 2: DEG down- and upregulated in neutrophil in absence of cDC1 analyzed by Bulk RNA seq**

| 12H | 12H | D2 | D2 | D4 | D4 |
|---|---|---|---|---|---|
| | | | | | |
| Genes down regulated | Genes up regulated | Genes down regulated | Genes up regulated | Genes down regulated | Genes up regulated |
| | | | | | |
| Ddx3y | Slfn4 | Ddx3y | Clec10a | Xist | Icosl |
| Eif2s3y | Lck | Eif2s3y | Cpa3 | Gbp2 | Mxd1 |
| Uty | Adora3 | Uty | Fcer1a | Tap1 | Cldn15 |
| Col3a1 | Bcam | Kdm5d | Kit | Irgm1 | Cyp4f18 |
| Sparc | Ggt1 | Cd207 | Kcnq1 | Gbp7 | Nqo1 |
| Col1a2 | Epn3 | Gm4017 | Serinc3 | Igtp | ll7r |
| Kdm5d | Aim1l | Ifi47 | Alox12e | Cd207 | Ndrg1 |
| Gpx3 | Gata3 | Gbp2 | Mybpc1 | Ifi47 | Hmox1 |
| Cxcl5 | ll12rb2 | Gm16867 | Ddc | Stat1 | Ampd3 |
| Col1a1 | Alox12e | 1520401A03Rik | Efemp1 | Nlrc5 | Hyal1 |
| Mmp3 | Smpdl3a | Il10 | Nr1d1 | Mpeg1 | Slc5a1 |
| Cd207 | Aifm2 | Rsad2 | Serpina3n | Gm16867 | Scube1 |
| Tnc | Myo1g | Col8a1 | Akr1c18 | Cxcl10 | Mical1 |
| Gm4017 | Limk2 | Mrgbp | Vcan | Uba7 | Slc5a8 |
| Col6a3 | Rsad2 | Igtp | Osmr | Gm1966 | Pfkl |
| Fbln2 | Ace | Oas3 | Cma1 | Gm15446 | Cpeb4 |
| Col5a1 | Nr1d1 | H2-M2 | Apod | Cd48 | Itgb3 |
| Serpinf1 | Alox8 | Zbp1 | Thbs2 | Gbp5 | Dusp13 |
| Mmp2 | Akr1c18 | P2rx2 | Tpsab1 | Gm12250 | Erol1l |
| Lox | Hr | Snx20 | C2 | Samhd1 | Scn8a |
| Ctgf | Ly6c2 | Oasl1 | Lpxn | Gbp3 | Prr13 |
| Stc1 | Ly6e | Impad1 | Got1 | Pafah2 | Abcg1 |
| Olfml3 | Arc | Jrk | Mmp19 | ligp1 | Lpin2 |
| Cxcl14 | Gcsam | Ppp2ca | Esyt1 | Fcgr4 | Tpsab1 |
| Dcn | Gpd1 | Plekhh3 | Cdh9 | P2rx2 | Map3k8 |
| Cpxm1 | Krt6b | Rac1 | Cd28 | Gm8995 | Afg3l2 |
| Mfge8 | Mal2 | Adgb | Scly | ll18bp | Slc16a3 |
| Chad | Ctsw | 9930022D16Rik | Marco | Ly6i | Dnase1l3 |
| Serpinhl1 | Cd7 | Dusp2 | Mrc1 | Fam26f | Cd163l1 |
| Sepp1 | Alas2 | 5730508B09Rik | Dpp7 | Irf9 | Slc40a1 |
| Cpz | Avil | Rnf149 | Vcam1 | Irgm2 | Ifi202b |
| Timp1 | Irf7 | Gpc1 | Egf | Fmod | Tbx19 |
| Fstl1 | Pkp1 | Stil | Ntrk1 | Clec4a3 | Selp |
| Bgn | Ube2l6 | Irgm1 | Atp8b5 | Gbp9 | Sele |
| Pcolce | Zbp1 | Abcc2 | Podn | Smo | Pfkfb3 |
| Igfbp7 | Gbp2 | Gm26686 | Htra3 | Erap1 | Smox |
| Col6a2 | Dnajc6 | Parp12 | Hsph1 | Sparc | Hdc |
| Arg1 | Pla2g2f | Dtx3l | Gpnmb | Herc6 | Bcl2l11 |
| Ctsk | Pitpnm2 | Igfbp3 | Anxa4 | Psmb10 | Procr |
| Pdpn | Oasl2 | Gbp7 | M1 ap | Abca8a | F3 |
| Adamts15 | Ephb6 | Arhgap20 | Slco2b1 | Clec4a1 | Ptgr1 |
| Pdgfrl | Usp18 | Tmem65 | Cyp11a1 | Irf1 | Ubap1 |
| Gm16867 | Far2 | Grip1 | Pdxk | Col6a2 | Plin2 |
| Fam102b | Itgal | Hapln1 | Asprv1 | Ngfr | Pdpn |
| Mpeg1 | Ccl22 | Gm15446 | Tpsb2 | Psmb9 | Spp1 |
| Bmp1 | Hephl1 | Gpr182 | Ccl7 | Tap2 | M1ap |
| Syt13 | Trim29 | Brca2 | Ccl2 | 2810468N07Rik | Prok2 |
| Aspn | Cd3e | Hgfac | C1qc | Akr1c18 | Calca |
| Ahrr | Acsbg1 | Grsf1 | C1qb | Gbp4 | Lat |
| Mmp14 | Cyp1a1 | Apol7a | F13a1 | Sema3g | Plet1 |
| Gfap | Ngp | Pde10a | Prune2 | Cnksr2 | Ngp |
| Timp2 | Oas2 | Gas1 | Tph1 | Zbtb7c | Ltf |
| Coch | Alox12b | Otud4 | Ctsg | Fam107a | Flcn |
| C7 | Rtp4 | Klf3 | Chil3 | Slamf8 | Alox12b |
| Serping1 | Evpl | Fst | Mgl2 | Tapbpl | Asprv1 |
| Has1 | Ifit1 | Irak1 | Serpinb1a | Reln | Rhov |
| 9930022D16Rik | Ptpn 13 | Gpt | Rell1 | Fgf11 | Plvap |
| Angptl7 | Ttc22 | Inhba | Pld4 | Sema4f | Egln3 |
| Apod | Dhcr24 | Gadd45a | Zfp658 | Psmb8 | Krt17 |
| Lum | Krt17 | Ly6i | Gstm1 | Ociad2 | Rsrp1 |
| Sectm1b | Isg15 | Plpp6 | Mcpt4 | Lama2 | Ackr1 |
| Nid1 | Tmem184a | Krt1 | Akap6 | Sv2c | Fgd3 |
| Ppic | Scd1 | Tgtp1 | Dok1 | Parp10 | Edem2 |
| Cilp | Prf1 | Rbl1 | Mrgprb1 | Col28a1 | Ciart |
| Alpl | Paqr7 | Mpeg1 | 4930449A18Rik | Cd163 | Echdc3 |
| Loxl4 | Zmat4 | Snx18 | Pisd-ps1 | Osgepl1 | Cdsn |
| Cdh11 | Arhgef4 | Ifit2 | Xist | Mapk15 | Slc26a11 |
| C1qtnf1 | Tapbpl | Sgol1 | Hspa1a | Phf24 | Klhdc4 |
| Fbxo27 | Cpa4 | Tbc1d22b | Ccer2 | Mrc2 | Dgkz |
| Gm15446 | Hlx | Csf3 | Tmem181b-ps | Lrba | Lat2 |
| Plpp3 | Kremen2 | ll1a | 5830416119Rik | Cd83 | Ccr6 |
| Antxr1 | Iffo2 | Slc35g1 | | Epha7 | Mmrn2 |
| Fuk | Elovl6 | Ttc39c | | Coch | Dsc1 |
| Col5a2 | Oasl1 | 2810029C07Rik | | Piezo2 | Lemd2 |
| Prkd1 | Gprin3 | ll23a | | Ccl9 | Insig1 |
| Chrna7 | Ifit2 | Msh6 | | Plpp2 | Tnfrsf26 |
| Mfap2 | Gjb2 | Yrdc | | Slfn8 | Ppp1 r3b |
| Slc13a3 | Fcgbp | Klf2 | | Sprn | Gpr68 |
| Inmt | Oxtr | | | Trpm1 | Cldn4 |
| Gm12258 | Cyp2g1 | | | Csf3 | Sprr1b |
| Reep3 | Sprr1a | | | Col1a2 | Sephs2 |
| Adamts2 | Fam78a | | | H2-T23 | Ftl1 |
| Ptx3 | Ffar2 | | | C77370 | Dedd2 |
| Scx | Pcdh1 | | | Batf2 | Ano9 |
| Kdelr3 | Dock8 | | | Psme2 | Spink5 |
| Fndc1 | Hbb-bs | | | Lypd6 | Kdm5d |
| Col6a1 | Oas1a | | | Tgfbr3 | Gstm1 |
| Rac1 | Slfn5 | | | Gm38252 | Sh2d3c |
| Pla2g2d | Ano9 | | | Synm | Dmkn |
| Igfbp6 | Fat2 | | | Col8a1 | Cd300lb |
| Hoga1 | H2-Q5 | | | Magi1 | Ldha |
| Efcab2 | Nod2 | | | Gm18301 | mt-Rnr1 |
| Tpst1 | Aak1 | | | Hsd17b12 | mt-Nd1 |
| Serpine2 | Adgrf2 | | | Farp2 | mt-Co1 |
| Wisp1 | H2-Q7 | | | Gdf3 | mt-Nd4 |
| Ptgfr | Krt79 | | | Zbp1 | mt-Nd5 |
| Sec16b | Ifit3b | | | Vstm5 | mt-Nd6 |
| Gfra1 | Ces2f | | | Cd180 | mt-Cytb |
| Fkbp7 | Parp10 | | | Cybb | Vps37b |
| Kdm8 | Cnfn | | | Kcnk2 | Tecpr1 |
| Ankrd28 | Krt2 | | | Dnph1 | H2-K2 |
| Zbtb16 | mt-Co1 | | | Dtx3l | Nipal1 |
| Penk | mt-Nd4 | | | Parp9 | ll2rb |
| Mrc1 | mt-Nd5 | | | | Uty |
| Adgrd1 | mt-Cytb | | | | Ddx3y |
| Ccl9 | Cyp2b19 | | | | Eif2s3y |
| Mfap4 | Oas1g | | | | Lsmem1 |
| Slc39a14 | ll2rb | | | | Wdfy1 |
| Gfpt2 | H2-Q6 | | | | Klhl21 |
| Gm42992 | Ifit3 | | | | Bglap3 |
| Gas1 | Ifi47 | | | | Thbd |
| Loxl3 | Gsdmc | | | | ll1bos |
| Pycr1 | Pglyrp2 | | | | Rab9 |
| P4ha3 | Esrp2 | | | | Rptoros |
| Scara5 | Xist | | | | Gm15832 |
| Plin5 | Ly6g6c | | | | Plcxd2 |
| Fkbp9 | Gm21887 | | | | Ly6g6c |
| Ccl7 | Erdr1 | | | | Mirt2 |
| Ptgis | Wdr46-ps | | | | Gm21887 |
| Cxcl12 | | | | | Tmem181b-ps |
| Ripk1 | | | | | Gm4017 |
| Mcoln2 | | | | | Gm37522 |
| Notum | | | | | 5830416119Rik |
| Rhoj | | | | | Gm44005 |
| F630028O10Rik | | | | | |
| Rdh10 | | | | | |
| Il7r | | | | | |
| Tshz3 | | | | | |
| Klf2 | | | | | |
| Pdgfra | | | | | |
| Sh3bgrl | | | | | |
| Lbp | | | | | |
| Fmod | | | | | |
| Tbx18 | | | | | |
| Fbn1 | | | | | |
| Gclm | | | | | |
| Zfp623 | | | | | |
| Gm29100 | | | | | |
| 3110099E03Rik | | | | | |
| Slc43a1 | | | | | |
| Syndig1 | | | | | |
| Arhgap20 | | | | | |
| Adam9 | | | | | |
| Plce1 | | | | | |
| Rcn3 | | | | | |
| Amigo1 | | | | | |
| Fst | | | | | |
| Fcrls | | | | | |
| Clptm1l | | | | | |
| Fzd1 | | | | | |
| Cd163 | | | | | |
| Lamp1 | | | | | |
| Sod3 | | | | | |
| Tubb3 | | | | | |
| Oaf | | | | | |
| Amfr | | | | | |
| Fgf23 | | | | | |
| Rnase4 | | | | | |
| Zfp558 | | | | | |
| Dab2 | | | | | |
| Cyp26b1 | | | | | |
| Pcdhga1 | | | | | |
| Fkbp10 | | | | | |
| Pf4 | | | | | |
| Eln | | | | | |
| Tm4sf1 | | | | | |
| Sgce | | | | | |
| Jam3 | | | | | |
| Eml1 | | | | | |
| Podn | | | | | |
| Gsn | | | | | |
| Lrrc48 | | | | | |
| Wnt5a | | | | | |
| Nkd2 | | | | | |
| Fads3 | | | | | |
| Cadm3 | | | | | |
| Apbb2 | | | | | |
| Cxcl1 | | | | | |

Taken together, these data indicate that dermal cDC1 promote neutrophil survival and, by consequence, skin inflammation associated with neutrophil accumulation.

### Single cell mRNA sequencing reveals two subsets of cDC1 in P. acnes-inflamed dermis

Multi-dimensional analyses are now revealing previously-unappreciated levels of functional heterogeneity within immune cell subsets (See et al., 2016), therefore the inventors applied single-cell mRNA sequencing to ask, at both the individual cell and population level, how dermal cDC1 might be mediating neutrophil recruitment and function.

The inventors first profiled the transcriptomic signature of 67 single dermal cDC1 isolated from the ears of WT mice, 16 hours after *P. acnes* injection, then used these data to group cells sharing distinct patterns of gene expression. Using the 67 single cell transcriptomes, the inventors performed Principal Component Analysis (PCA) (data not shown), followed by hierarchical clustering of the 400 most variably-expressed genes using Fluidigm SINGuLAR and SPIN (See Methods) (Szulwach et al., 2015, Tsafrir et al., 2005). The SINGuLAR approach revealed two distinct cell clusters within the 67 cell cDC1 population: a minor cluster of 8 cells (Subset 1) and a major cluster of 59 cells (Subset 2) (data not shown). The same clusters, comprised of the same individual cells, were identified by the SPIN analysis (data not shown).

Supervised hierarchical clustering using the SINGuLAR method identified 1796 DEGs between the two dermal cDC1 clusters (**Table 5**), of which 240 were less highly expressed and 1556 were more highly expressed in Subset 1 vs Subset 2 (p value ≤ 0.05).

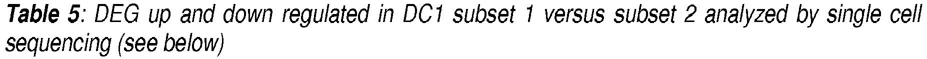

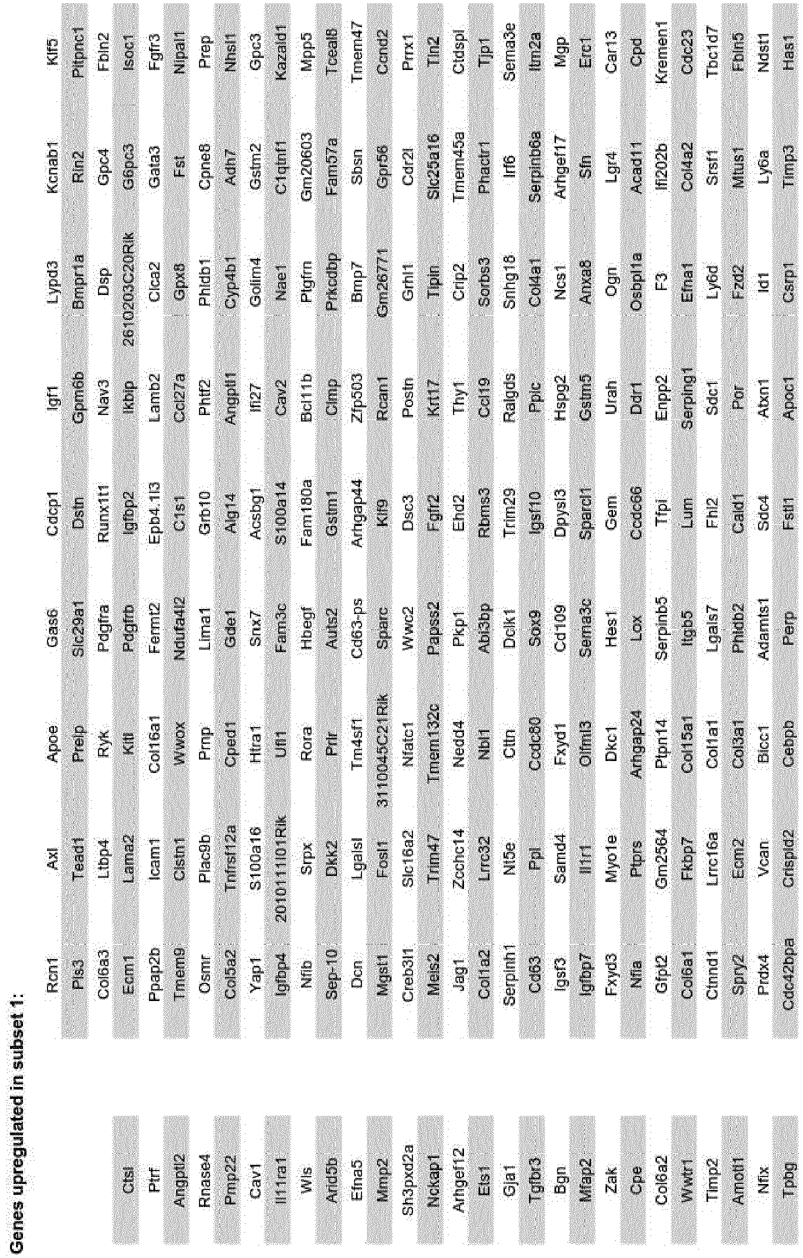

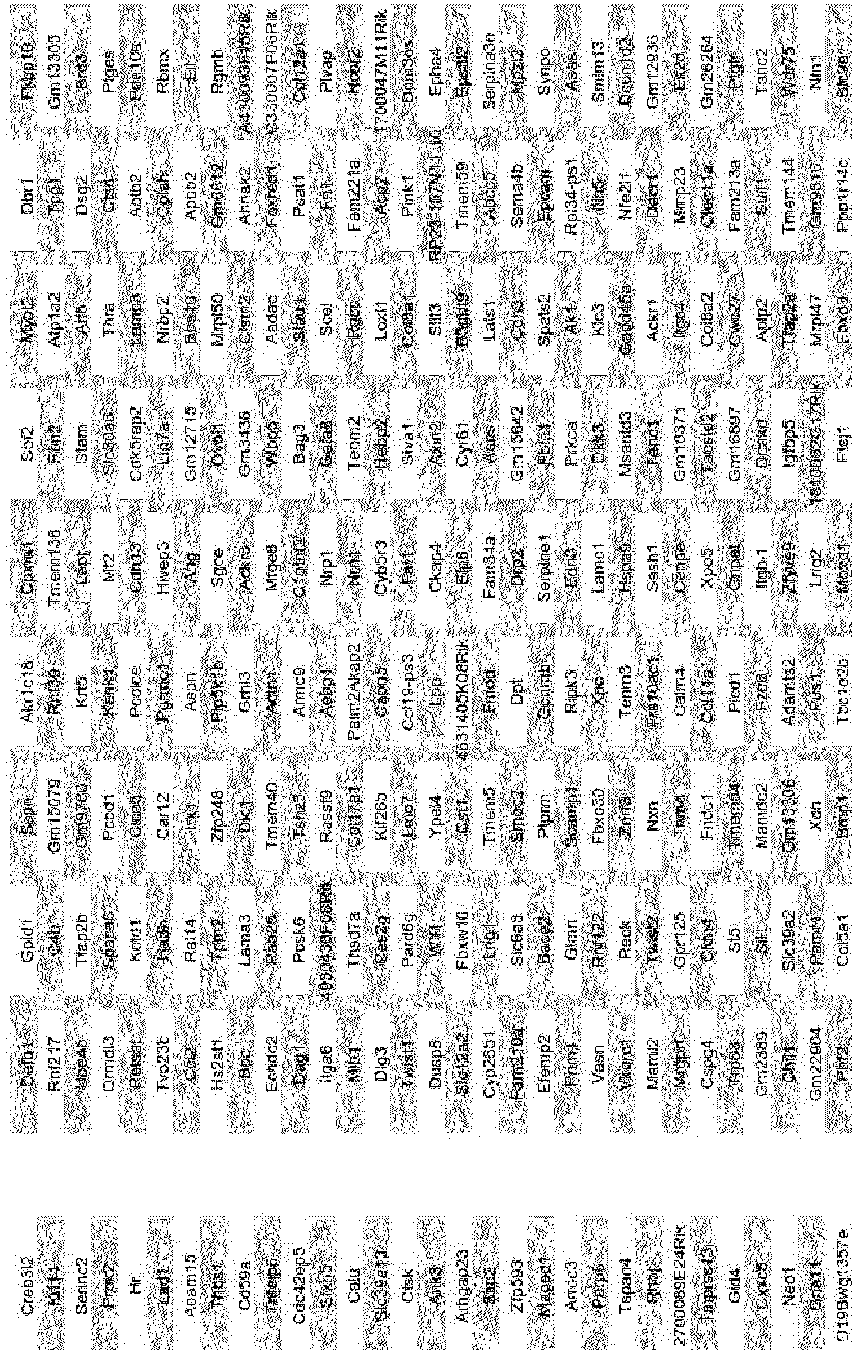

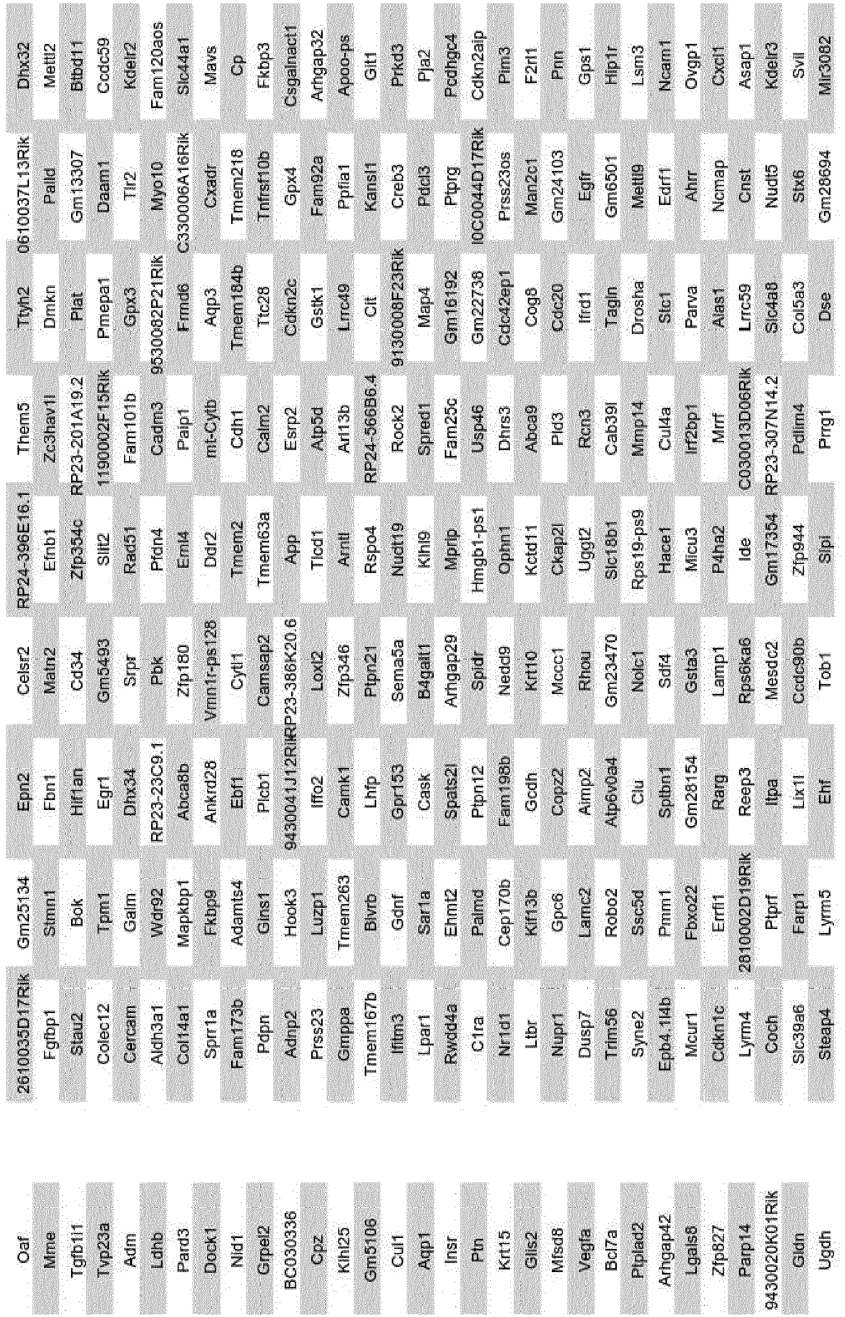

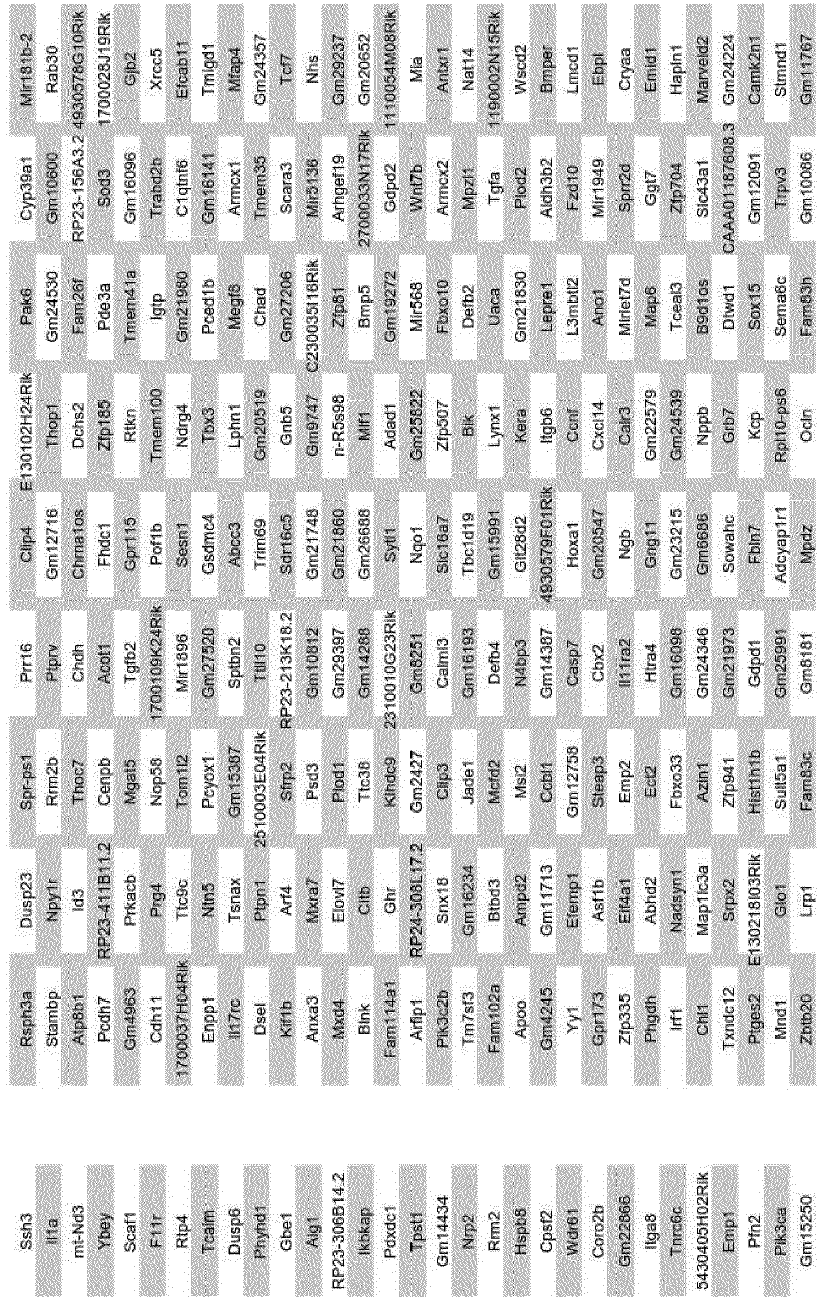

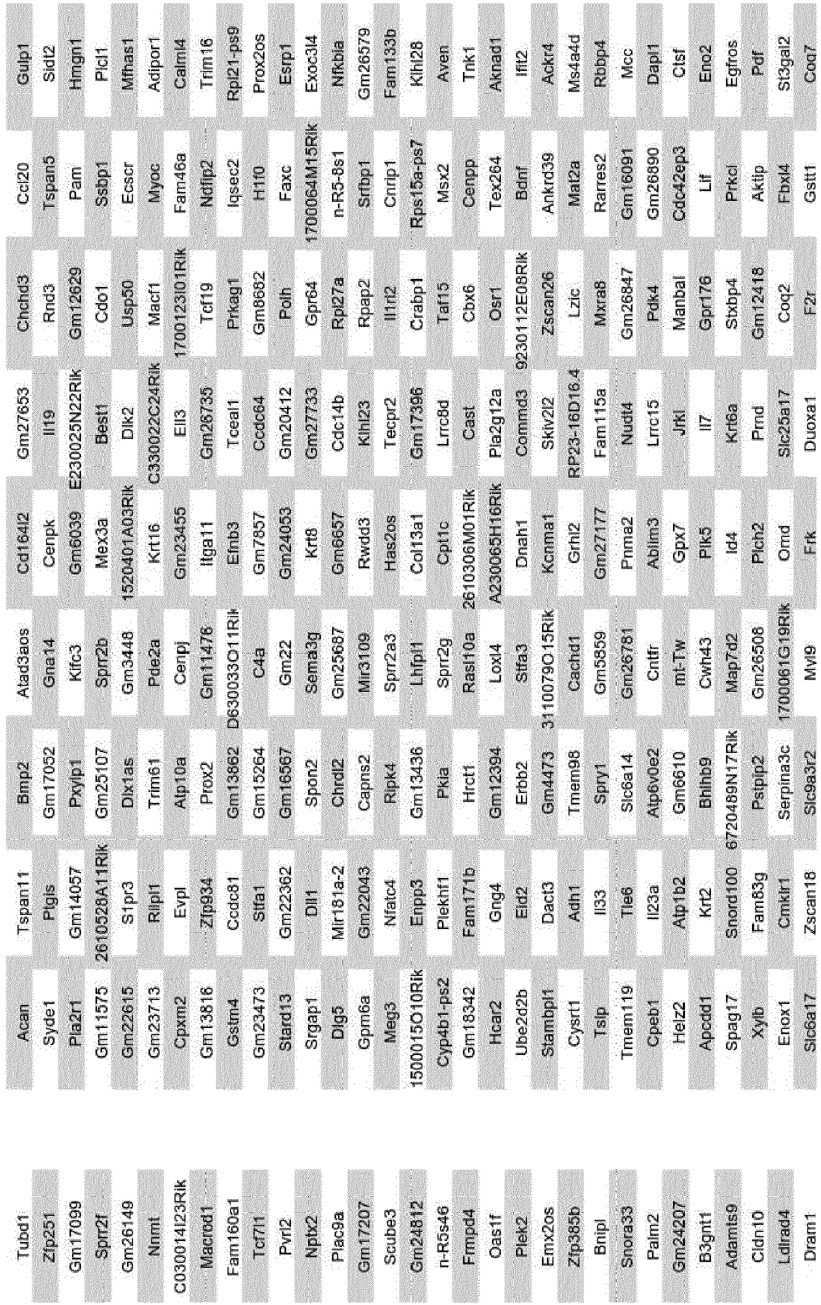

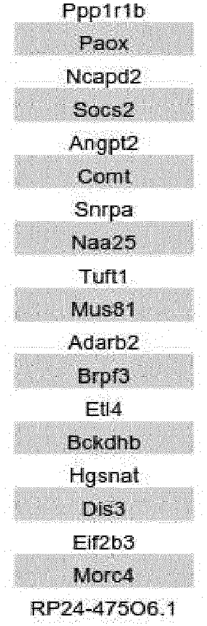

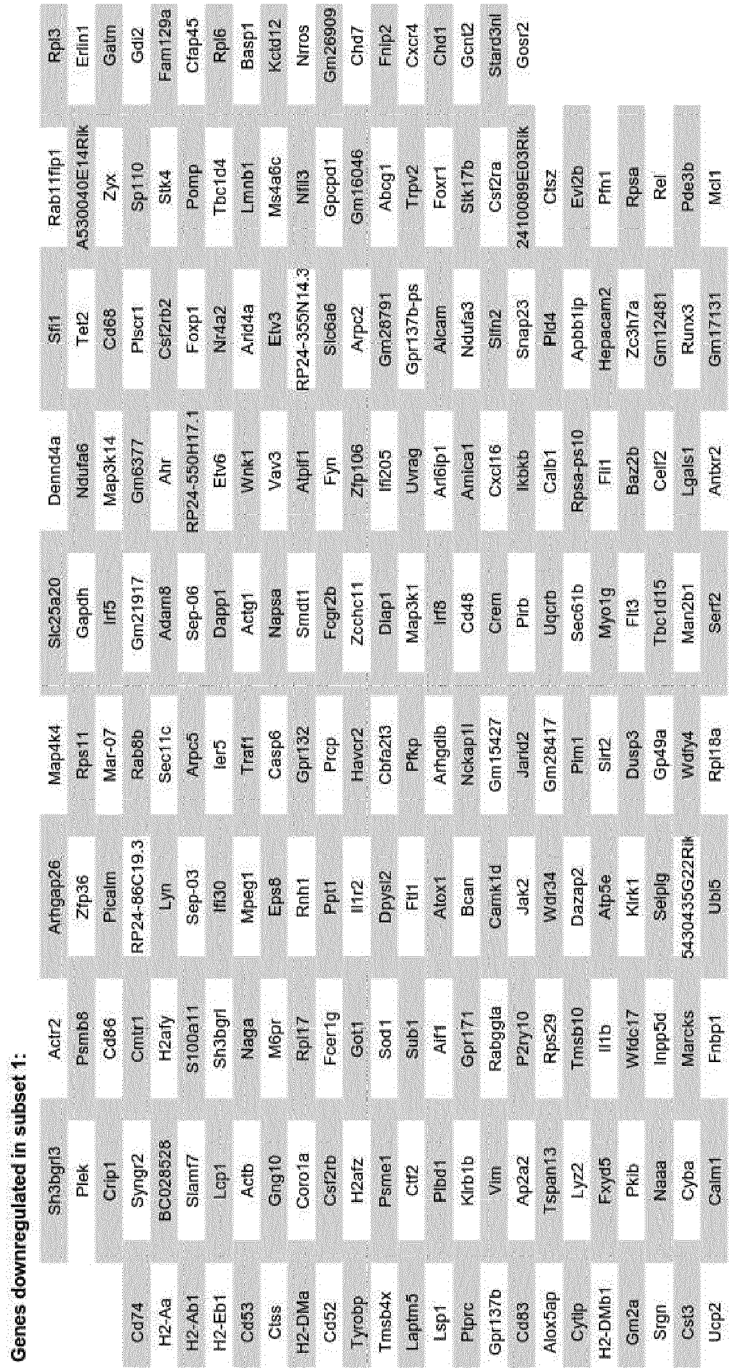

IPA revealed that most of the DEGs expressed more abundantly in the minor "Subset 1" were related to DC immune function after capture of antigen, activation, phagocytosis, priming and migration pathways; specifically genes related to DC migration to the lymph node, maturation and activation during immune response and also to antigen priming and presentation by MHC-I (data not shown). Interestingly, some genes related to immune cell recruitment were also expressed more highly in the minor Subset 1: genes related to monocyte/macrophage recruitment, including *ccl2, cxcl14* and *csf-1* (Lu et al., 1998, Qian et al., 2011, Lu et al., 2016); or linked to T cell activation, such as *cc120* and *ccl27a* (Hirota et al., 2007, Woodland and Kohlmeier, 2009) (data not shown). Among the genes less highly expressed in Subset 1, the inventors found those involved in the major histocompatibility complex class II (MHCII) presentation pathway (data not shown). Altogether, this analysis suggests that the minor subset of cDC1 represents an activated DC population in *P*. acnes-injected skin.

The inventors then asked whether these cDC1 clusters could be retrospectively identified in skin based on differences in expression of membrane proteins encoded by DEGs highlighted in the single cell transcriptome dataset. The inventors first confirmed by flow cytometry that the transcript abundance for the surface markers EpCAM, CD59 and Ly6D differed significantly in the two cDC1 subsets (data not shown). Next, the inventors used flow cytometry to measure the frequency and intensity of cell-surface protein level expression of these markers across the dermal cDC1 population from both inside and outside the granulomatous area of *P. acnes*-or PBS- injected skin. This confirmed the presence of the minor population of cDC1 expressing high levels of CD59, EpCAM and Ly6D (i.e. "the EpCAM⁺CD59⁺Ly6D⁺ cDC1 subset"), which was enriched inside the granuloma (data not shown), suggesting a link between the presence of the bacteria and the increased expression of these markers by cDC1. Lastly, the inventors confirmed that these markers were specific to cDC1 and not shared by cDC2 (data not shown). Thus an activated dermal cDC1 subset ("the EpCAM⁺CD59⁺Ly6D⁺ cDC1 subset") can be distinguished within the cDC1 population at both the transcriptional and surface marker level.

### Activated dermal cDC1 express neutrophil chemo-attractant VEGFα in the presence of P. acnes

The inventors then screened the single-cell cDC1 gene expression dataset for secreted molecules that could play a role in neutrophil recruitment and activation. Multiple potential neutrophil-modulatory candidates were expressed at a significantly higher level in the activated minor cDC1 subset, including *cxcl1, il22, il23α, il19, il7, il1α and vegfα* (data not shown). As VEGF/VEGFR regulate neutrophil infiltration into tissues during infection (Christoffersson et al., 2012, Massena et al., 2015), the inventors asked whether cDC1 produced this protein in response to *P. acnes.* It is not possible to isolate sufficient numbers of activated dermal cDC1 for intracellular flow cytometry analysis of cytokine production, so the inventors instead derived cDC1 from bone marrow cells (Mayer et al., 2014) and exposed them to *P. acnes in vitro.* Bone marrow-derived cDC1 produced increasing amounts of VEGFα in response to escalating densities of the bacterium and to similar levels than after Toll-like receptor (TLR) agonists, except the agonist of TLR7/8 (CLO75) (data not shown).

To understand the relevance of these pathways in our model, the inventors tested the expression profile of VEGF receptors and other markers on neutrophils infiltrating the skin in the presence or absence of cDC1. Using the neutrophil-specific CyTOF panel (**Table 6**), the inventors uncovered marked changes in bone marrow, blood and skin neutrophil phenotype in response to *P. acnes.*

**Table 6: Table of antibodies used for mass cytometry analysis listing metal conjugate, antibody clone name, supplier**

| **Isotope tag** | **Sequence** | **Clone/Company** |
|---|---|---|
| Qdot (Cd112/114) | CD19 | 6D5 (Invitrogen) |
| Pd--102 | *Barcode* | |
| Pd--104 | *Barcode* | |
| Pd---105 | *Barcode* | |
| Pd--106 | *Barcode* | |
| Pd--108 | *Barcode* | |
| Pd--110 | *Barcode* | |
| Ln--113 | *Barcode* | |
| Ln--115 | CD90 | 53-2.1 (Biolegend) |
| La--139 | CD45 | 30--F11 (Biolegend) |
| Nd--141 | CD43 | eBioR2/60 (Ebioscience) |
| Nd--142 | MHC class II | Y3P (BioXcell) |
| Nd--143 | CD49D | R1-2 (Ebioscience) |
| Nd--144 | CD11a | FD441.8 (BD) |
| Nd--145 | CD64 | X54-5/7.1 (Biolegend) |
| Sm--147 | Ly6G | IA8 (BD) |
| Nd--148 | Ly6c | HK1.4 (Biolegend) |
| Nd--150 | CD54 | YNI.7.4 (BioXCell) |
| Eu--151 | CD62L | MEL14 (Biolegend) |
| Sm--152 | CD127 | SB/199 (Biolegend) |
| Eu--153 | CD11b | M1/70 (BD) |
| Sm--154 | CD3 | 145.2C11 (BD) |
| Gd--155 | CD86 | GL7 (BD) |
| Gd--156 | CCR1 | S15040E (Biolegend) |
| Gd--157 | CXCR2 | TG11/CXCR2 (Biolegend) |
| Gd--158 | IL17R | MAB4481 (R&Dsystem) |
| Tb--159 | F4/80 | C1:A3-1 (Serotec) |
| Gd--160 | TNFR | 55R-28 6(Biolegend) |
| Dy--161 | CX3CR1 | ab10400 (Abcam) |
| Dy--162 | PDL--1 | 10F.9G2 (Biolegend) |
| Dy--163 | VEGFR1 | BAF471 (R&Dsystem) |
| Dy--164 | CD103 | 2e7 (Biolegend) |
| Ho--165 | CCR5 | HM-CCR5 (Biolegend) |
| Er--166 | CCR2 (a--APC) | CCR2 APC -- 475301 (R&D systems) |
| | | a--APC -- APC003 (Biolegend) |
| Er--167 | CXCR4 | 2B11 (Ebioscience) |
| Tm--169 | IL33R | DIH9 (Biolegend) |
| Er---170 | VEGFR2 | 89B3A5 (Biolegend) |
| Yb--172 | CD48 | HM48-1 (Biolegend) |
| Yb--173 | SIRPa (CD172a) | P84 (BD) |
| Yb--174 | CD24 | M1/69 (Biolegend) |
| Lu--175 | CD44 | IM7 (Ebioscience) |
| Yb--176 | CD49B | DX5 (Biolegend) |
| Ir--191/193 | DNA | DVS Sciences |
| 195 | CisPlatin | Sigma Aldrich |
| | *live*/*dead* | |
| Cell length | *Cell length* | N/A |

Bone marrow neutrophils upregulated overtime CXCR2 and CD62L expression, two markers known to regulate the neutrophil release from bone marrow to the blood circulation (Eash et al., 2010, Martin et al., 2003). Blood neutrophils expressed high levels of CXCR2 associated with an upregulation of the expression of CD62L and CD49d, two markers associated to the tethering and rolling along postcapillary venules, prior to transmigration across the vascular endothelium to sites of inflammation (Rogowski et al., 1998, Furze and Rankin, 2008, Scheiermann et al., 2015) (data not shown). Importantly, whereas in both control and depleted mice neutrophils gradually increased CD44 and PDL1 expression, the frequency and intensity of VEGFR1 expression was only significantly increased in controls, measured by both CYTOF and flow cytometry. These effects were specific to neutrophils in the skin of cDC1-depleted mice, as comparable levels and patterns of marker expression were observed in bone marrow and blood neutrophils in control and depleted animals (data not shown).

To understand the functional impact of VEGFα on early recruitment and regulation of VEGFR1 expression by neutrophils, the inventors neutralized this cytokine *in vivo* using a specific blocking antibody. After concomitant intra-dermal injection of WT mice with anti-VEGFα antibodies and *P. acnes,* the inventors measured significant reductions in ear swelling and neutrophil infiltration compared to control WT mice, to levels comparable with mice lacking cDC1 and LC (LG-DTR 24 hours post-DT) (data not shown). Accordingly, intradermal injection of recombinant VEGFα protein concomitant with *P. acnes* reinstated the inflammatory response in mice depleted of cDC1 and LC (Langerin DTR mice 24H post DT injection) to the same extent as in WT mice (data not shown). Together, these data identify a key role for cDC1-derived VEGFα in neutrophil recruitment to the site of *P. acnes* injection in mice.

### cDC1-derived VEGFα is necessary for the inflammatory response to P. acnes

The transcription factors CREB, STAT3 and HIF-α, regulate VEGFα expression by *in vitro* generated DCs (Salvi et al., 2016). Hence, the inventors used a Cre/flox strategy in which HIF-α expression was specifically absent in cDC. The inventors generated CD45.2 CD11c-cre⁻ or CD11c-cre⁺ HIF^{flox} into CD45.1 WT B6 chimeras to specifically ablate expression of HIF in dermal cDC and not in radioresistant LC, without affecting their development and abundance (data not shown).
Following intra-dermal *P. acnes* injection, the absence of HIF-α expression in cDC led to significantly reduced ear swelling, and significantly less neutrophil and Ly-6C^{high} monocyte infiltration (data not shown), compared to the HIF-α-sufficient CD11c-cre⁻ mice. The absence of HIF-α in cDC prevented the emergence of the activated cDC1 subset as the phenotype of activated cDC1 as the frequency and intensity of cell-surface protein level expression of EpCAM, CD59 and Ly6D after *P. acnes* injection was similar to the PBS control (data not shown). These data suggest a key role for HIF-α for dermal cDC1 activation and their subsequent VEGFα secretion *in vivo* and demonstrate the central role of cDC1-derived VEGFα in controlling the immune response to *P. acnes* infection.

## Claims

1. A VEGF inhibitor for use for preventing and/or treating acne.

2. The VEGF inhibitor for use according to claim 1, wherein acne includes all acne forms, especially simple acne, comedogenic acne, papulopustular acne, papulocomedonic acne, nodulocystic acne, acne conglobata, cheloid acne of the nape of the neck, recurrent miliary acne, necrotic acne, neonatal acne, occupational acne, acne rosacea, senile acne, solar acne, medication-related acne and acne vulgaris.

3. The VEGF inhibitor for use according to claim 1 or 2, wherein it is an inhibitor of VEGF expression, a VEGF receptor tyrosine kinase inhibitor, or an inhibitor of VEGF interaction with its target sequences, particularly its receptor(s).

4. The VEGF inhibitor for use according to any one of claims 1 to 3, wherein it is a VEGF-A inhibitor.

5. The VEGF inhibitor for use according to any one of claims 1 to 4, wherein it is an inhibitor of VEGF expression chosen from siRNA and antisense oligonucleotides.

6. The VEGF inhibitor for use according to any one of claims 1 to 4, wherein it is an inhibitor of VEGF interaction with its receptor(s) chosen from chemical molecules, peptides, proteins and antibodies.

7. The VEGF inhibitor for use according to any one of claims 1 to 4, wherein it is an inhibitor of VEGF interaction with its receptor(s) chosen from bevacizumab, ranibizumab and ramucirumab.

8. The VEGF inhibitor for use according to any one of claims 1 to 4, wherein it is a VEGFR tyrosine kinase inhibitor.

9. The VEGF inhibitor for use according to claim 8, wherein it is a VEGFR tyrosine kinase inhibitor chosen from:
Sorafenib
Sunitinib
Pazopanib
Vandetanib
Axitinib
Cediranib
Cabozantinib
Foretinib
Tivozanib
Lenvatinib
Lucitanib
Dovitinib
Orantinib
Nintedanib
Vatalanib
Telatinib
Motesanib
3-((quinolin-4-ylmethyl)amino)-N-(4-(trifluoromethoxy)phenyl)thiophene-2-carboxamide Brivanib alaninate
(3R,4R)-4-amino-1-((4-((3-methoxyphenyl)amino)pyrrolo[2,1-f][1,2,4]triazin-5-yl)methyl)piperidin-3-ol
Linifanib
6-[4-[(4-Ethylpiperazin-1-yl)methyl]phenyl]-N-[(1R)-1-phenylethyl]-7H-pyrrolo[2,3-d]pyrimidin-4-amine
N-(5-((2-(cyclopropanecarboxamido)imidazo[1,2-b]pyridazin-6-yl)oxy)-2-methylphenyl)-1,3-dimethyl-1 H-pyrazole-5-carboxamide
13-isobutyl-4-methyl-10-(pyrimidin-2-ylamino)-1,2,4,7,8,13-hexahydro-6H-indazolo[5,4-a]pyrrolo[3,4-c]carbazol-6-one
3-((4-bromo-2,6-difluorobenzyl)oxy)-5-(3-(4-(pyrrolidin-1-yl)butyl)ureido)isothiazole-4-carboxamide, and
Regorafenib.

10. The VEGF inhibitor for use according to any one of claims 1 to 9, wherein it is formulated in a pharmaceutical composition, preferably a topical pharmaceutical composition.

11. The VEGF inhibitor for use according to claim 10, wherein the pharmaceutical composition is in liquid, pasty or solid form and, more particularly, in the form of ointments, aqueous, aqueous-alcoholic or oily solutions, dispersions of the optionally two-phase lotion type, serum, aqueous, anhydrous or lipophilic gels, powders, soaked pads, syndets, wipes, sprays, foams, sticks, shampoos, compresses, washing bases, emulsions of liquid or semi-liquid consistency such as milk, obtained by dispersing a fatty phase in an aqueous phase (O/W) or inversely (W/O), a microemulsion, suspensions or emulsions of soft, semi-liquid or solid of white or colored cream type, gel or ointment, suspensions of microspheres or nanospheres or lipid or polymeric vesicles, or microcapsules, micro- or nanoparticles or of polymeric or gelled patches allowing a controlled release.

12. The VEGF inhibitor for use according to any one of claims 1 to 11, wherein it reduces the recruitment of neutrophils, or it prevents and/or decreases the inflammatory response to *P.acnes* bacterium.

13. The VEGF inhibitor for use according to any one of claims 1 to 12, wherein it prevents and/or decreases VEGF-R1 expression on neutrophils induced by cDC1, particularly by the subset of EpCAM⁺CD59⁺Ly6D⁺ cDC1.
